Hoechst

EPA EPO-OEB
DG 1
Reçu:
10-11-1988
02

Hoechst AG · Postfach 80 03 20 · D-6230 Frankfurt am Main 80

Hoechst Aktiengesellschaft
Zentrale Patentabteilung
Postfach 80 03 20 · Brüningstraße 50
6230 Frankfurt am Main 80
Telefon: (0 69) 3 05-0 · Telex: 4 1 234 700 ho d
Telegramm: hoechstag frankfurtmain
Fax: infotec (0 69) 35 71 75
Dresdner Bank AG, Frankfurt am Main 80
(BLZ 500 800 00) Kto. Nr. 7 355 555 00
Commerzbank AG, Frankfurt am Main 80
(BLZ 500 400 00) Kto. Nr. 2570729
Deutsche Bank AG, Frankfurt am Main 1
(BLZ 500 700 10) Kto. Nr. 926006
Hessische Landesbank — Girozentrale —
Frankfurt am Main 1
(BLZ 500 500 00) Kto. Nr. 24100 000
Landeszentralbank in Hessen, Frankfurt am Main 1
(BLZ 500 000 00) Kto. Nr. 500 08190
Postgiroamt Frankfurt am Main 1
(BLZ 500 100 60) Kto. Nr. 1442-605

An das
EUROPÄISCHE PATENTAMT
Erhardtstraße 27
8000 München 2

EPA-EPO-OEB
MÜNCHEN
Empfang bestätigt
Receipt acknowledged
Accusé reception
AB

| Ihre Zeichen | Ihre Nachricht vom | Unsere Zeichen | Telefon Durchwahl (0 69) 3 05- | Frankfurt am Main |
|---|---|---|---|---|
| | | Dr.WS/Ba | 6243 | 01. November 1988 |

Europäische Patentanmeldung Nr. 88113055.3

- HOE 87/F 242 JK -

Datum 11/11/88

Es wird ferner um die Korrektur folgender offensichtlicher Fehler gebeten:

Seite 3, Zeile 34 und Seite 47, Zeile 21 : "(2f + 1-q)" muß jeweils "(2p + 1-q)" lauten (vgl. Seite 5, Zeile 36).

./.

Hoechst

| Empfänger | Unsere Zeichen | Datum | Blatt |
| --- | --- | --- | --- |
| Europäisches Patentamt<br>8000 München 2 | Dr.WS/Ba | 1. November 1988 | -2- |

Europäische Patentanmeldung Nr. 88113055.3
- HOE 87/F 242 JK -

Seite 31, Zeile 1: "5-Chlor-2-picolin" ist durch "Beispiel 1" zu ersetzen.

Seite 35, Zeile 21: "m-Chlorbenzoesäure" sollte korrigiert werden in "m-Chlorperbenzoesäure".

HOECHST AKTIENGESELLSCHAFT

i.V. Schmidt

Dr. Becker Dr. Schmidt

Anlagen

900 2 012

(19) Europäisches Patentamt
European Patent Office
Office européen des brevets

(11) Veröffentlichungsnummer: **0 304 732 A2**

(12) # EUROPÄISCHE PATENTANMELDUNG

(21) Anmeldenummer: **88113055.3**

(22) Anmeldetag: **11.08.88**

(51) Int. Cl.4: **C07D 495/04 , A61K 31/44 , //(C07D495/04,333:00,235:00)**

Patentansprüche für folgenden Vertragsstaat: ES + GR.

Ein Antrag gemäss Regel 88 EPÜ auf Berichtigung liegt vor. Über diesen Antrag wird im Laufe des Verfahrens vor der Prüfungsabteilung eine Entscheidung getroffen werden (Richtlinien für die Prüfung im EPA, A-V, 2.2).

(30) Priorität: **15.08.87 DE 3727244**
**24.12.87 DE 3744022**
**19.05.88 DE 3816996**

(43) Veröffentlichungstag der Anmeldung: **01.03.89 Patentblatt 89/09**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Lang, Hans-Jochen, Dr.**
**Rüdesheimer Strasse 7**
**D-6238 Hofheim am Taunus(DE)**
Erfinder: **Weidmann, Klaus, Dr.**
**Talweg 11**
**D-6242 Kronberg/Taunus(DE)**
Erfinder: **Rippel, Robert, Dr.**
**Frankfurter Strasse 66**
**D-6238 Hofheim am Taunus(DE)**
Erfinder: **Scheunemann, Karl-Heinz, Dr.**
**Geisenheimer Strasse 88**
**D-6000 Frankfurt am Main 71(DE)**
Erfinder: **Herling, Andreas W., Dr.**
**Am Walberstück 5**
**D-6277 Bad Camberg(DE)**

(54) **Substituierte Thienoimidazol-Derivate, Verfahren zu ihrer Herstellung, sie enthaltende pharmazeutische Zubereitungen und ihre Verwendung als Magensäuresekretionshemmer.**

(57) Die Erfindung betrifft Verbindungen der Formel I

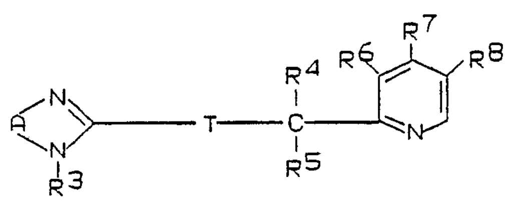

(I)

in welcher

A für a) 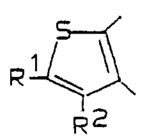 , b) 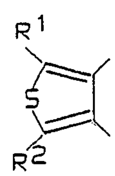 oder c) 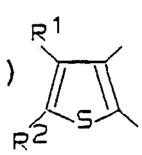 steht,

T -S-, -SO- oder $-SO_2-$ bedeutet, $R^3$ Wasserstoff, Alkanoyl, Alkylcarbamoyl, Alkoxycarbonyl, Benzyloxycar-

EP 0 304 732 A2

bonyl oder eine andere physiologisch verträgliche Schutzgruppe bedeutet, $R^4$ und $R^5$ für Wasserstoff oder Alkyl stehen, $R^6$ und $R^8$ Wasserstoff, Halogen, Alkyl oder einen Substituenten der Formel $-O-[CH_2]o-CpH_{(2p+1-q)}Hal_q$ bedeuten und $R^7$ Wasserstoff, (subst.)Alkyl, (subst.)Alkoxy, $-O-[CH_2]_o-C_pH_{(2p+1-q)}Hal_q$, $NR'R''$, Alkylmercapto, Alkylsulfinyl oder Alkylsulfonyl ist, ein Verfahren zu deren Herstellung, diese enthaltene pharmazeutische Präparate und ihre Verwendung als Arzneimittel.

## Substituierte Thienoimidazol-Derivate, Verfahren zu ihrer Herstellung, sie enthaltende pharmazeutische Zubereitungen und ihre Verwendung als Magensäuresekretionshemmer

Thienoimidazol-Derivate mit magensäuresekretionshemmender Wirkung sind aus den EP-A1-234 485, EP-A2-201 094 und EP-A-237 248 bekannt.

Die vorliegende Erfindung betrifft neue Thienoimidazol-Derivate der Formel I

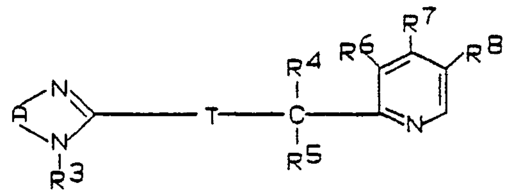

(I)

in welcher

A für a) 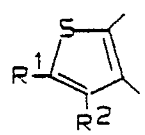 , b) 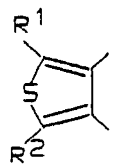 oder c) 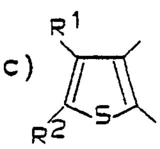 steht,

T -S-, -SO- oder $-SO_2-$ bedeutet,

$R^1$ und $R^2$ gleich oder verschieden sind und Wasserstoff, Halogen, Cyano, Nitro, Trifluormethyl, $(C_1-C_6)$-Alkyl, $(C_1-C_6)$-Hydroxyalkyl, $(C_1-C_6)$-Alkoxy, $-O-[CH_2]_o-C_pH_{(2p+1-q)}Hal_q$, vorzugsweise $(C_1-C_8)$-Fluoralkoxy, $(C_1-C_8)$-Fluoralkenyloxy, $(C_1-C_8)$-Fluoralkinyloxy, $-OCF_2Cl$ oder $-O-CF_2-CHFCl$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, N-$(C_1-C_4)$-Alkylsulfamoyl oder N,N-Di-$(C_1-C_4)$-alkylsulfamoyl bedeuten, oder, falls A wie oben unter (a) oder (c) definiert ist, auch gemeinsam $-[CH_2]_n-$ oder -CH=CH-CH=CH- bedeuten können, wobei eine $CH_2$-Gruppe gegebenenfalls durch $NR'$, O, S, SO oder $SO_2$ ersetzt ist, oder einen substituierten $(C_6-C_{12})$-Aryloxy-, $(C_7-C_{11})$-Aralkyloxy, $(C_6-C_{12})$-Aryl- oder $(C_7-C_{11})$-Aralkyl-Rest bedeuten, der im Arylteil 1, 2, 3, 4 oder 5 gleiche oder verschiedene Substituenten aus der Reihe Halogen, Cyano, Nitro, Trifluormethyl, $(C_1-C_6)$-Alkyl, $(C_1-C_6)$-Alkoxy, $-O-[CH_2]_o-C_pH_{(2p+1-q)}Hal_q$, $-OCF_2Cl$, $-O-CF_2-CHFCl$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, $NR'R''$, Sulfamoyl, N-$(C_1-C_4)$-Alkylsulfamoyl oder N,N-Di-$(C_1-C_4)$-alkylsulfamoyl trägt, bedeuten,

$R^3$ Wasserstoff, $(C_1-C_6)$-Alkanoyl, $(C_1-C_6)$-Alkylcarbamoyl, $(C_1-C_6)$-Alkoxycarbonyl, Benzyloxycarbonyl oder eine andere physiologisch verträgliche, vorzugsweise im sauren Medium und/oder unter physiologischen Bedingungen abspaltbare $N^{im}$-Schutzgruppe wie z. B. $(C_1-C_{10})$-Acyloxy-$(C_1-C_6)$-alkyl, vorzugsweise $(C_1-C_{10})$-Alkanoyloxy-$(C_1-C_6)$-alkyl, Benzoyloxy-$(C_1-C_6)$-alkyl, Benzyloxycarbonyloxy-$(C_1-C_6)$-alkyl oder $(C_1-C_6)$-Alkoxycarbonyloxy-$(C_1-C_6)$-alkyl bedeutet,

$R^4$ und $R^5$ gleich oder verschieden sind und Wasserstoff oder $(C_1-C_3)$-Alkyl bedeuten,

a) $R^6$ Wasserstoff bedeutet und

$R^8$ Fluor, Chlor oder Brom bedeutet, oder

b) $R^6$ $(C_1-C_3)$-Alkyl bedeutet und

$R^8$ Fluor bedeutet, oder

c) $R^6$ Fluor oder Brom bedeutet und

$R^8$ Wasserstoff bedeutet, oder

d) $R^6$ Fluor bedeutet und

$R^8$ $(C_1-C_3)$-Alkyl bedeutet, oder

e) einer der Substituenten $R^6$ und $R^8$ den Rest $-O-[CH_2]_o-C_pH_{(2p+1-q)}Hal_q$ bedeutet und der andere Wasserstoff, Halogen, $(C_1-C_3)$-Alkyl oder den Rest $-O-[CH_2]_o-C_pH_{(2p+1-q)}Hal_q$ bedeutet, oder

f) einer der Substituenten $R^6$ und $R^8$ einen substituierten $(C_6$-$C_{12})$-Aryloxyrest oder $(C_7$-$C_{11})$-Aralkyloxyrest bedeutet, der im Arylteil 1, 2, 3, 4 oder 5 gleiche oder verschiedene Substituenten aus der Reihe Halogen, Cyano, Nitro, Trifluormethyl, $(C_1$-$C_6)$-Alkyl, $(C_1$-$C_6)$-Alkoxy, -O-$[CH_2]_o$-$C_pH_{(2p-1-q)}Hal_q$, -$OCF_2Cl$, -O-$CF_2$-CHFCl, $(C_1$-$C_6)$-Alkylmercapto, $(C_1$-$C_6)$-Alkylsulfinyl, $(C_1$-$C_6)$-Alkylsulfonyl, $(C_1$-$C_6)$-Alkylcarbonyl, $(C_1$-$C_6)$-Alkoxycarbonyl, Carbamoyl, N-$(C_1$-$C_4)$-Alkylcarbamoyl, N,N-Di-$(C_1$-$C_4)$-alkylcarbamoyl, $(C_1$-$C_6)$-Alkylcarbonyloxy, $(C_3$-$C_8)$-Cycloalkyl, Sulfamoyl, N-$(C_1$-$C_4)$-Alkylsulfamoyl oder N,N-Di-$(C_1$-$C_4)$-alkylsulfamoyl trägt, bedeutet, und der andere Wasserstoff, Halogen, $(C_1$-$C_3)$-Alkyl, den Rest -O-$[CH_2]_o$-$C_pH_{(2p+1-q)}Hal_q$ oder einen substituierten $(C_6$-$C_{12})$-Aryloxyrest oder $(C_7$-$C_{11})$-Aralkyloxyrest bedeutet, Hal Halogen, beispielsweise Fluor, Chlor, Brom, vorzugsweise Fluor, bedeutet,

$R^7$ Wasserstoff, Halogen, $(C_1$-$C_{12})$-Alkyl, $(C_1$-$C_{12})$-Alkoxy, -O-$[CH_2]_o$-$C_pH_{(2f+1-q)}Hal_q$, -NR′R″, $(C_1$-$C_{12})$-Alkoxy-$(C_1$-$C_{12})$-alkyl, $(C_1$-$C_{12})$-Alkoxy-$(C_1$-$C_{12})$-alkoxy, $(C_7$-$C_{11})$-Aralkyloxy, $(C_1$-$C_{12})$-Alkylmercapto, $(C_1$-$C_{12})$-Alkylsulfinyl oder $(C_1$-$C_{12})$-Alkylsulfonyl bedeutet, oder

falls mindestens einer der Substituenten $R^6$ und $R^8$ einen substituierten $(C_6$-$C_{12})$-Aryloxyrest oder $(C_7$-$C_{11})$-Aralkyloxyrest bedeutet, so kann $R^7$ ebenfalls einen wie vorstehend unter f) definierten substituierten $(C_6$-$C_{12})$-Aryloxyrest oder $(C_7$-$C_{11})$-Aralkyloxyrest bedeuten,

$R^5$ und $R^6$ gemeinsam für -$[CH_2]_i$- stehen,

R′ und R″ gleich oder verschieden sind und Wasserstoff, $(C_6$-$C_{12})$-Aryl oder $(C_1$-$C_{12})$-Alkyl bedeuten oder

R′ und R″ gemeinsam für -$[CH_2]_h$- stehen, worin eine $CH_2$-Gruppe durch O, S, N-$(C_1$-$C_4)$-Alkanoylimino oder N-$(C_1$-$C_4)$-Alkoxylcarbonylimino ersetzt sein kann,

h = 3, 4, 5 oder 6, vorzugsweise 4 bis 6 ist,

i = 1, 2, 3 oder 4, vorzugsweise 1 bis 3 ist,

n = 3 oder 4 ist,

o = 0, 1, 2 oder 3, vorzugsweise 0 oder 1 ist,

p = 1, 2, 3, 4, 5, 6, 7 oder 8, vorzugsweise 1 bis 5 ist und

q = 0 oder 1 bis (2p + 1) ist,

sowie deren physiologisch verträgliche Salze, mit der Maßgabe, daß die Verbindungen

5-Methyl-2-(2-pyridylmethylthio)-3H-thieno[2,3-d]imidazol,
5-Methyl-2-((4-methoxy-2-pyridyl)methylthio)-3H-thieno[2,3-d]imidazol,
2-(Pyridyl)methylthio-3H-thieno[2,3-d]imidazol,
2-((4-Methoxy-2-pyridyl)methylthio)-3H-thieno[2,3-d]imidazol,
5-Acetyl-2-((4-methoxy-3,5-dimethyl-2-pyridyl)methylthio)-3H-thieno-[2,3-d]imidazol,
2-((4-Methoxy-3,5-dimethyl-2-pyridyl)methylthio)-3H-thieno[2,3-d]imidazol-5-carbonsäuremethylester,
5-Acetyl-2-((4-methoxy-2-pyridyl)methylthio)-3H-thieno[2,3-d]imidazol,
5-Acetyl-2-(2-pyridyl)methylthio-3H-thieno[2,3-d]imidazol,

sowie die entpsrechenden Sulfinyl-Verbindungen ausgenommen sind.

1H-Thieno[3,4-d]imidazol-Derivate der Formel I, worin A wie oben unter (b) definiert ist, sind bevorzugt. T ist vorzugsweise eine -SO-Gruppe.

Besonders bevorzugt sind Verbindungen der Formel I, worin

A vorzugsweise wie oben unter (b) definiert ist,

T vorzugsweise eine -SO-Gruppe bedeutet,

$R^1$ und $R^2$ gleich oder verschieden sind und Wasserstoff, $(C_1$-$C_3)$-Alkyl, Halogen, $(C_1$-$C_4)$-Alkoxy oder $(C_1$-$C_4)$-Alkoxycarbonyl bedeuten,

$R^3$ wie oben definiert ist,

$R^4$ und $R^5$ jeweils Wasserstoff bedeuten,

$R^6$ und $R^8$ gleich oder verschieden und wie vorstehend unter a) - e) definiert sind und

$R^7$ Wasserstoff, $(C_1$-$C_3)$-Alkyl oder einen halogenierten Alkoxy-, Alkenyloxy- oder Alkinyloxy-Rest der Formel -O-$[CH_2]_oC_pH_{(2p+1-q)}Hal_q$, $(C_1$-$C_7)$Alkoxy, Benzyloxy oder $(C_1$-$C_7)$-Alkoxy-$(C_1$-$C_3)$-alkyl bedeutet, und wobei Halogen vorzugsweise Fluor, Chlor oder Brom bedeutet, o 0 oder 1, p 1 bis 8 und q 0 oder 1 bis (2p + 1) sind, und

insbesondere aber Verbindungen der Formel I, worin

A vorzugsweise wie oben unter (b) definiert ist,

T vorzugsweise eine -SO-Gruppe bedeutet,

$R^1$ und $R^2$ gleich oder verschieden sind und Wasserstoff oder $(C_1$-$C_3)$-Alkyl bedeuten,

$R^3$ wie oben definiert ist,

$R^4$ und $R^5$ jeweils Wasserstoff bedeuten,

$R^6$ und $R^8$ gleich oder verschieden und wie vorstehend unter a) - e) definiert sind und

$R^7$ Wasserstoff, $(C_1$-$C_4)$-Alkoxy, einen fluorierten n-Alkoxyrest der Formel -O-$[CH_2]_o$-$C_pH_{(2p+1-q)}Hal_q$ oder $(C_1$-$C_3)$-Alkyl bedeutet, wobei Halogen Fluor bedeutet, o 1, p 1 bis 5 und q 0 oder 1 bis (2p + 1) sind.

Von besonderer Bedeutung sind
2-[3-Methoxy-4-(2,2,2-trifluorethoxy)-2-picolylsulfinyl]-1H-thieno[3,4-d]imidazol,
2-[3-Methoxy-4-(2,2,3,3-tetrafluorpropoxy)-2-picolylsulfinyl]-1H-thieno[3,4-d]imidazol,
2-[3-Methoxy-4-(2,2,3,3,3-pentafluorpropoxy)-2-picolylsulfinyl]-1H-thieno[3,4-d]imidazol,
2-[3-Methoxy-4-(2,2,3,3,4,4,4-heptafluorbutoxy)-2-picolylsulfinyl]1H-thieno[3,4-d]imidazol,
2-(3-Brom-4-methoxy-2-picolylsulfinyl)-1H-thieno[3,4-d]imidazol,
2-(4-Methoxy-5-brom-2-picolylsulfinyl)-1H-thieno[3,4-d]imidazol,
2-(4-Methoxy-5-chlor-2-picolylsulfinyl)-1H-thieno[3,4-d]imidazol.

Alkyl und davon abgeleitete Reste wie beispielsweise Alkoxy, Alkylmercapto, Alkylsulfinyl, Alkylsulfonyl, Aralkyl oder Alkanoyl können geradkettig oder verzweigt sein.

($C_6$-$C_{12}$)-Aryl ist beispielsweise Phenyl, Naphthyl oder Biphenyl, bevorzugt ist Phenyl.

($C_7$-$C_{11}$)-Aralkyl ist beispielsweise Benzyl oder Phenethyl, vorzugsweise Benzyl. Entsprechendes gilt für davon abgeleitete Reste wie Aralkyloxy.

Halogen steht für Fluor, Chlor, Brom oder Jod.

Geeignete $N^{im}$-Schutzgruppen $R^3$ sind z.B. im Zusammenhang mit substituierten Picolylsulfinylbenzimidazolen in EP-A-176 308 und EP-A2-221 041, für Thienoimidazolverbindungen in EP-A-234 485 beschrieben.

Bevorzugte $N^{im}$-Schutzgruppen sind solche, die in Gegenwart von Säuren, vorzugsweise in einem pH-Bereich von etwa 1 - 6 und/oder unter physiologischen Bedingungen abgespalten werden können.

Gegebenenfalls vorhandene chirale C- und S-Atome können sowohl in der R- als auch in der S-Konfiguration vorkommen. In solchen Fällen liegen Verbindungen der Formel I in Form der reinen Enantiomeren oder als Stereoisomerengemisch (wie Enantiomerengemisch und Diastereomerengemisch) vor.

Als Salze kommen insbesondere Alkali- und Erdalkalisalze und Salze mit physiologisch verträglichen Aminen in Frage.

Die Erfindung betrifft weiterhin ein Verfahren zur Herstellung von Verbindungen der Formel I, das dadurch gekennzeichnet ist, daß man

a) Verbindungen der Formel II

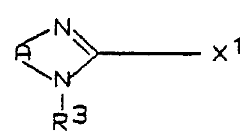

(II)

in welcher A, $R^1$, $R^2$ und $R^3$ wie oben definiert sind und
$X^1$ i. eine Abgangsgruppe oder
ii. -SH, $-S^-M^+$ oder $-SO_2^-M^+$ bedeutet,
umsetzt mit Verbindungen der Formel III

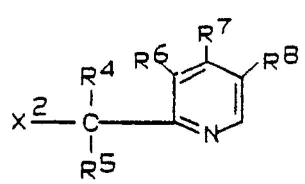

(III)

in welcher $R^4$, $R^5$, $R^6$, $R^7$ und $R^8$ wie oben definiert sind und
$X^2$ im oben genannten Fall i. -SH, $-S^-M^+$ oder $-SO_2^-M^+$ und
im oben genannten Fall ii. vorzugsweise eine Abgangsgruppe oder OH bedeutet, oder

b) Verbindungen der Formel IV,

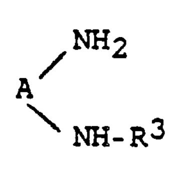

(IV)

in welcher A, $R^1$, $R^2$ und $R^3$ wie oben definiert sind,

umsetzt mit Verbindungen der Formel V

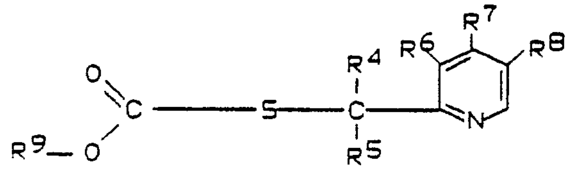

(V)

in welcher $R^4$, $R^5$, $R^6$, $R^7$ und $R^8$ wie oben definiert sind und $R^9$ für eine veresternde Gruppe steht,
i. in Verbindungen der Formel I (eine) gegebenenfalls vorhandene -S-Gruppe(n) gewünschtenfalls zu(r) -SO- oder $-SO_2$-Gruppe(n) oxidiert,
ii. in Verbindungen der Formel I (eine) gegebenenfalls vorhandene -SO-Gruppe(n) gewünschtenfalls zu(r) $-SO_2$-Gruppe(n) oxidiert,
iii. Verbindungen der Formel I, worin $R^3$ für Wasserstoff steht, gewünschtenfalls acyliert, alkyliert oder aralkyliert,
iv. Verbindungen der Formel I, worin $R^3$ nicht Wasserstoff bedeutet, gewünschtenfalls verseift und
v. Verbindungen der Formel I gewünschtenfalls in ihre physiologisch verträglichen Salze überführt,
wobei zwei oder mehr der Maßnahmen i.-iv. auch in einer anderen als der angegebenen Reihenfolge ausgeführt werden können.

$M^+$ steht für Kationen wie beispielsweise Alkali-, Erdalkali-, Ammonium- oder Alkylammonium-Ionen, insbesondere Natrium-oder Kalium-Ionen.

Setzt man gemäß der hier bevorzugten Verfahrensvariante (a) Verbindungen der Formel II mit Verbindungen der Formel III um, so steht $X^1$ oder $X^2$ für eine Abgangsgruppe, die nucleophil ablösbar ist, wie Cl, Br, J, $-O-SO_2-CH_3$, $-O-SO_2-CF_3$ oder $-O-SO_2-(C_6H_4-pCH_3)$.

Die Umsetzung einer Verbindung der Formel II mit einer Verbindung der Formel III oder deren Salzen erfolgt in einem inerten Lösungsmittel wie z.B. Wasser, Methylenchlorid, Methanol, Ethanol, Aceton, Essigsäureethylester, Toluol, Tetrahydrofuran, Acetonitril, Dimethylformamid, Dimethylacetamid, Dimethylsulfoxid oder Gemischen dieser Lösungsmittel zweckmäßigerweise in Gegenwart einer anorganischen oder organischen Base, wie z.B. Natrium-oder Kaliumhydroxid, -carbonat, -alkoxid, -hydrid, -amid, Ammoniak, Triethylamin, Tributylamin, Pyridin bei -20 bis +150 °C, vorzugsweise bei 0 - 80 °C.

Die Verbindungen der Formel II sind bekannte Verbindungen (siehe z.B. Gronowitz, "The Chemistry of Heterocyclic Compounds", Band 44, "Thiophene and its Derivatives", Parts 1-3, New York 1985-6) oder können in Analogie zu bekannten Verfahren hergestellt werden z.B. durch Ringschluß entsprechend substituierter 2,3-, 3,4- oder 4,5-Diaminothiophene der oben definierten Formel IV mit entsprechenden Schwefelverbindungen wie Schwefelkohlenstoff (z.B. DE-A-31 32 167).

Die hierfür benötigten 2,3-, 3,4- oder 4,5-Diaminothiophene sind entweder literaturbekannt oder können in Analogie zu bekannten Verfahren hergestellt werden. Sie werden z.B. durch Reduktion entsprechend substituierter Aminonitrothiophene erhalten.

In den bei Verfahrensvariante (b) eingesetzten Estern der Formel V steht $R^9$ für eine veresternde Gruppe, vorzugsweise $(C_1-C_6)$-Alkyl oder Benzyl.

Die Umsetzung einer Verbindung der Formel IV mit einer Verbindung der Formel V gemäß Verfahrensvariante (b) erfolgt analog der in Preston et al., Benzimidazoles and Congeneric Tricyclic Compounds, Part 1, New York, Seiten 10-13 beschriebenen Verfahrensweisen.

Die so erhaltenen Verbindungen der Formel I können, falls $R^3$ Wasserstoff bedeutet, in physiologisch verträgliche Salze umgewandelt werden.

Verbindung der Formel I mit T = -S- können ferner mit geeigneten Oxidationsmitteln in solche mit T = -SO- oder $-SO_2-$ umgewandelt werden. In gleicher Weise lassen sich auch -S-Gruppen in den Substituenten $R^1$, $R^2$ und $R^7$ oxidieren.

Diese Reaktion erfolgt in einem geeigneten, inerten Lösungsmittel wie z.B. Methylenchlorid, Chloroform, Tetrachlorkohlenstoff, 1,2-Dichlorethan, Toluol, Essigsäureethylester, Essigsäure, Trifluoressigsäure, Wasser, Methanol, Ethanol oder Gemischen derselben bei -20 °C bis +150 °C, vorzugsweise bei -10 °C bis +40 °C.

Als Oxidationsmittel kommen z.B. in Betracht:
Wasserstoffperoxid, Persäuren und Perester wie Peressigsäure, Trifluorperessigsäure, Monoperphthalsäure, m-Chlorperbenzoesäure und deren Ester, Ozon, Distickstofftetroxid, Jodosobenzol, N-Chlorsuccinimid, 1-Chlorbenzotriazol, Natriumhypochlorit, Natriumbromit, Kaliumperoxodisulfat, t-Butylhypochlorit, Tetrabutylammoniumperjodat oder -permanganat, Natrium-meta-perjodat, Selen- oder Mangandioxid, Cerammonnitrat, Chromsäure, Chlor, Brom, Diazabicyclo[2.2.2]octan-Bromkomplex, Dioxandibromid, Pyridiniumperbromid, Sulfurylchlorid, 2-Arylsulfonyl-3-aryloxaziridine, Titantetraisopropylat/tert. Butylhydroperoxid (gegebenenfalls unter Zusatz von Dialkylestern der (D)- bzw. (L)-Weinsäure und einer definierten Menge Wasser).

Ebenso können isolierte, ggf. immobilisierte oxidierende Enzyme oder Mikroorganismen als Oxidationsmittel Anwendung finden.

Die Oxidationsmittel werden in äquimolaren Mengen, ggf. auch in einem geringen Überschuß von 5 - 10 Mol-% bei der Oxidation zu T = -SO- oder auch in größerem Überschuß und/oder bei höherer Reaktionstemperatur eingesetzt, wenn eine Oxidation zu T = $-SO_2-$ gewünscht wird.

Die Erfindung betrifft weiterhin neue Zwischenprodukte der Formel III. Sie können nach dem Fachmann bekannten Methoden wie sie beispielsweise in "The Chemistry of Heterocyclic Compounds - Pyridine and its Derivatives", Pts. 2 and 3, E. Klingsberg Ed. Interscience Publishers, 1962 beschrieben sind, hergestellt werden.

Neben der Synthese der Verbindungen der Formel III, wobei $X^2$ eine Abgangsgruppe bedeutet, aus Verbindungen der Formel III, worin $X^2$ eine Hydroxygruppe bedeutet, können die Verbindungen der Formel III, in der $X^2$ Chlor oder Brom bedeuten, z.B. durch Halogenierung der entsprechenden 2-Picoline mit N-Bromsuccinimid, Trichlorisocyanursäure (Chem. Ber. 120, 649-651 (1987)) oder anderen N-Halogenamiden wie N-Chlorphthalimid hergestellt werden.

Ohne die Erfindung auf die folgenden Beispiele zu begrenzen, sind nachstehend einige Herstellungsverfahren für Verbindungen der Formel III, in denen $X^2$ Hydroxy bedeutet, beschrieben. Deren Umwandlung in Verbindungen der Formel III, in denen $X^2$ eine Abgangsgruppe bedeutet, erfolgt nach Standardmethoden.

Verbindungen der Formel III, in denen
$X^2$ eine Hydroxygruppe, einer der Substituenten $R^6$ und $R^8$ Brom, der andere Wasserstoff bedeutet, können nach Schema 1 erhalten werden.

Ein Gemisch der Verbindungen der Formel VII/VIII wird durch Bromierung von 2-Picolin nach bekannten Verfahren (vgl. z.B. Bull. Soc. Chim. France 2466, 1972) erhalten.

Nach N-Oxidation z.B. mit m-Chlorperbenzoesäure und Nitrierung werden die Verbindungen IX und X durch Kristallisation bzw. Chromatographie aufgetrennt und rein hergestellt.

Die Verbindungen der Formeln XI und XIII, in denen $R^7$ Alkoxy bedeutet, werden jeweils aus den Verbindungen der Formeln IX und X und den entsprechenden Alkoholaten $MR^7$ erhalten, wobei M wie auf Seite 9 definiert ist, und z.B. mit Acetanhydrid oder $(CF_3CO)_2O$ und anschließender Verseifung der Acetate zu den Verbindungen der Formeln XII und XIV umgesetzt.

**Schema 1:**

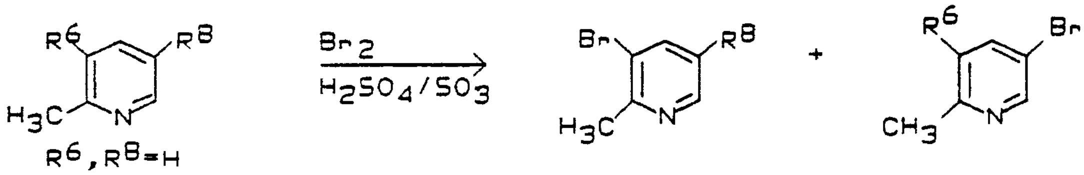


(VI) (VII) (VIII)

VII/VIII

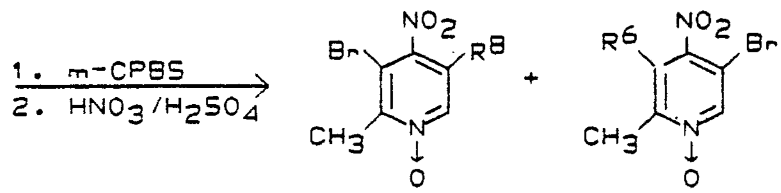


(IX) (X)

IX

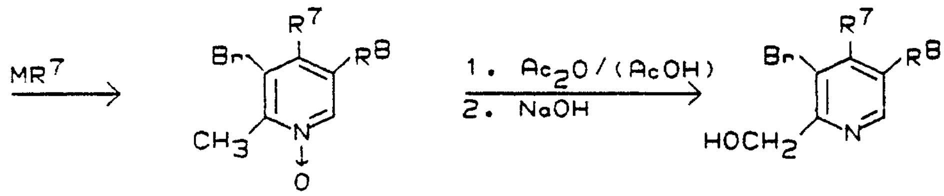


(XI) (XII)

X

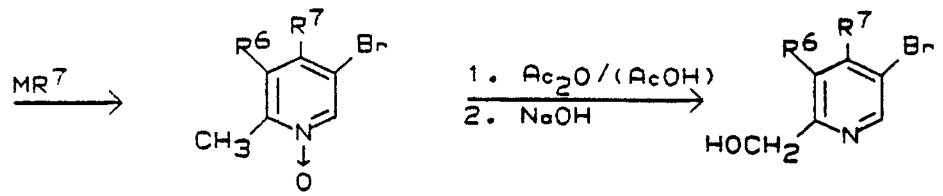


(XIII) (XIV)

Im Schema 2 ist die Synthese für Verbindungen der Formel III, in denen $X^2$ eine Hydroxygruppe, $R^6$ Wasserstoff und $R^8$ Chlor bedeutet, dargestellt. Das nach dem bekannten Verfahren über Abbaureaktionen von Carbonsäurederivaten der 6-Methylnicotinsäure erhältliche 5-Amino-2-picolin XV (vgl. J. Prakt. Chem. 133 [1932] 19) wird nach dem Fachmann geläufigen Reaktionen (Sandmeyer-Reaktion, N-Oxidation, Nitrierung, Reaktion mit Alkoholaten, Umlagerung mit Acetanhydrid, Acetatverseifung) zu Verbindungen der Formel XX umgesetzt.

**Schema 2:**

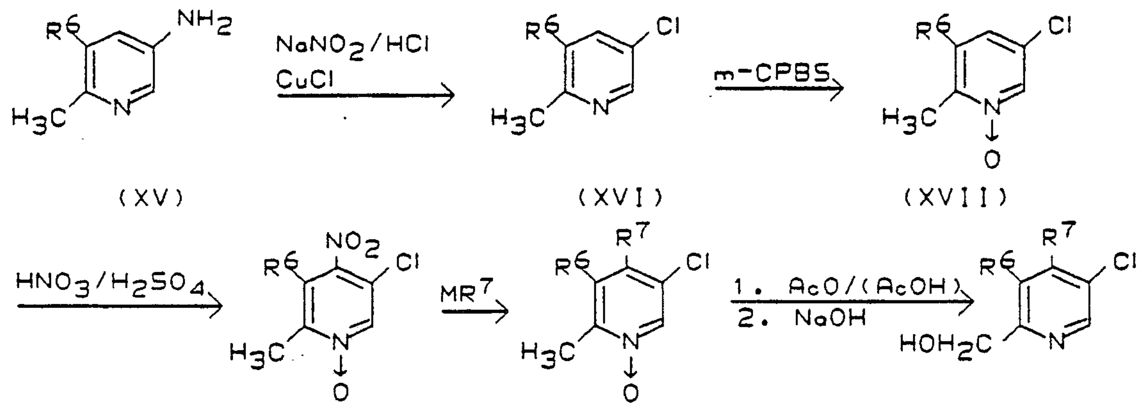


(XV) (XVI) (XVII)

(XVIII) (XIX) (XX)

Verbindungen der Formel III, in denen $X^2$ eine Hydroxygruppe, $R^6$ Wasserstoff oder Methyl und $R^8$ Fluor bedeuten, können, wie in Schema 3 gezeigt, erhalten werden.

Unter den Bedingungen der Ullmann-Reaktion können die Verbindungen der Formel VIII in 5-Amino-2-picoline XV überführt werden (vgl. Rec. Trav. Chim. 84 (7), 951-64 (1965)).

Über Abbaureaktionen von Carbonsäurederivaten der 6-Methylnicotinsäure der Formel XXI werden ebenfalls die Verbindungen XV zugänglich. Die Synthese der Fluorderivate XXII erfolgt zweckmäßig unter den Bedingungen der Schiemann-Reaktion, wie für XXII ($R^6$ = H) bekannt (vgl. J. Med. Chem. 13, 1124 (1970)).

Die 4-Nitro-5-fluor-2-picoline der Formel XXIII werden, wie für die analogen 3-Fluorisomeren bekannt, zu den Aminoderivaten XXIV reduziert (vgl. Rocz. Chem, 41 (2), 279-87 (1967)) und in die Dihalogenide XXV überführt.

**Schema 3: $R^6$ = H, $CH_3$**

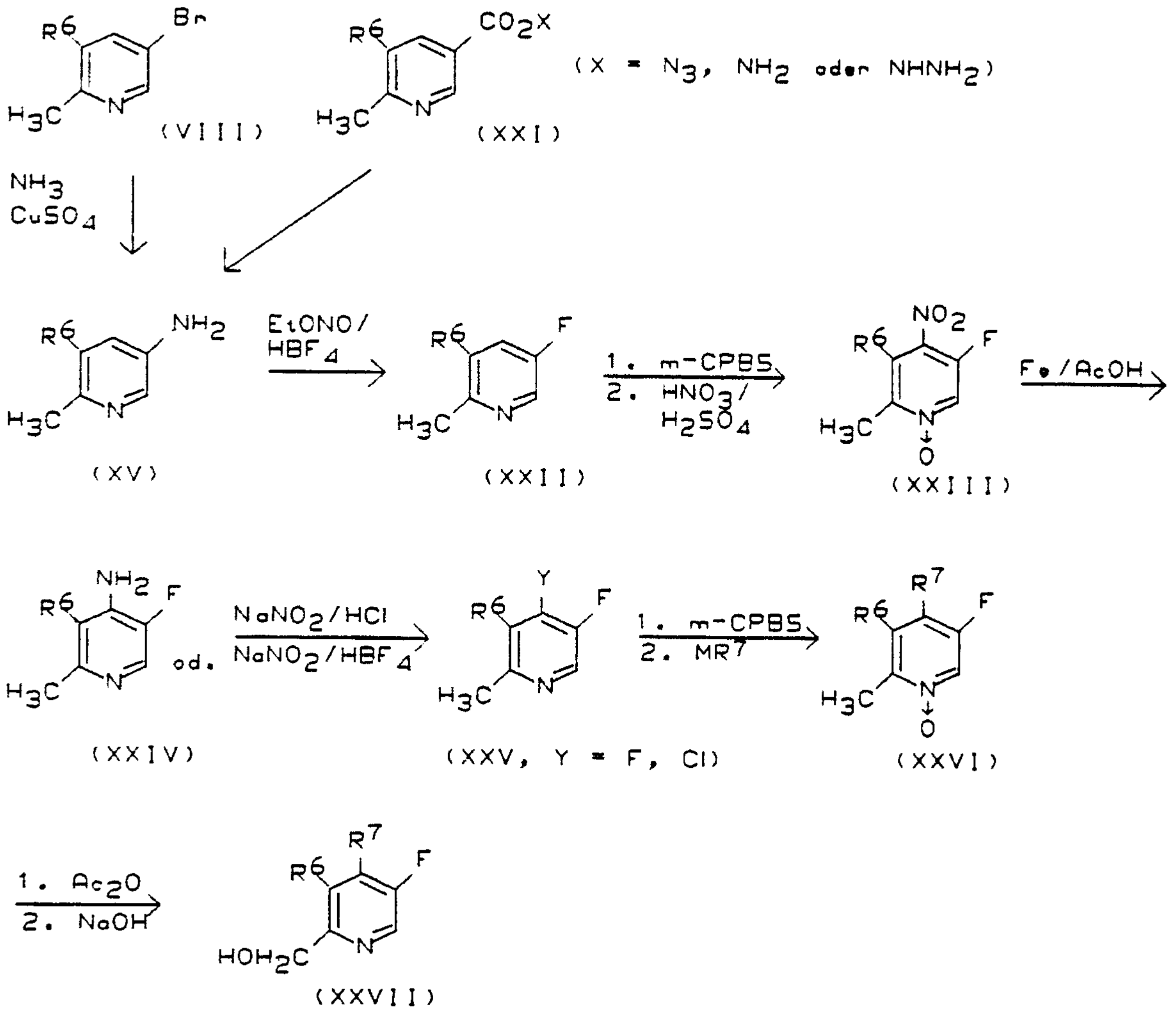


Nach N-Oxidation, Substitution des 4-Halogens durch Alkoholat $R^7$ und Umlagerung, vorzugsweise mit Acetanhydrid oder Trifluoracetanhydrid und Acetatverseifung werden die Picolylalkohole der Formel XXVII erhalten.

In analoger Weise können die entsprechenden Verbindungen mit $R^6$ = Fluor und $R^8$ = H oder ($C_1$-$C_3$)-Alkyl hergestellt werden.

Verbindungen der Formel III, in denen $X^2$ eine Hydroxygruppe bedeutet, und sowohl $R^6$ als auch $R^8$ Fluor oder Chlor bedeuten, können z.B. nach Schema 4 gewonnen werden:

**Schema 4:**

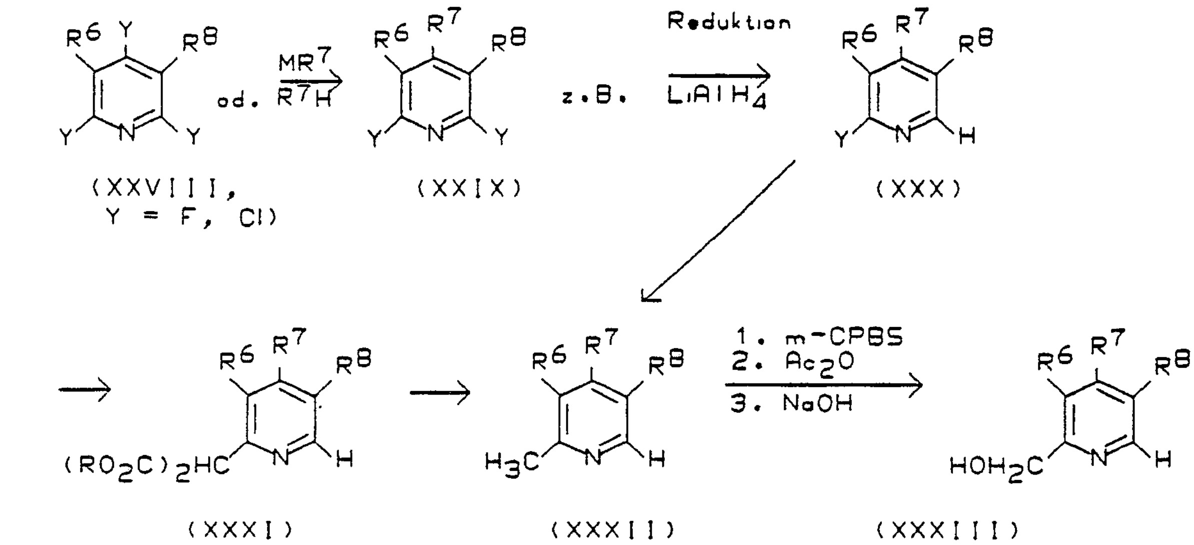


(XXVIII, Y = F, Cl) (XXIX) (XXX) (XXXI) (XXXII) (XXXIII)

Substitutionsreaktionen mit Nucleophilen wie z.B. Aminen und Alkoholaten führen von den Halogenderivaten XXVIII zu Verbindungen der Formel XXIX, deren Reduktion XXX ergibt, vgl. J. Chem. Soc. 1965, 575. Durch Umsetzung von Verbindungen der Formel XXX mit Salzen von Malonsäurediestern können Ester der Formel XXXI erhalten werden, die nach saurer oder alkalischer Verseifung im sauren Medium zu den substituierten 3,5-Dihalogen-2-picolinen der Formel XXXII decarboxylieren.

Auch können die Verbindungen der Formel XXX mit metallorganischen Reagenzien wie z.B. Methylmagnesiumhalogeniden oder Methyllithium direkt zu XXXII umgesetzt werden.

Durch nucleophile Substitutionen mit $C_1$-Synthonen wie z.B. Salze von Dithianen oder Cyaniden können aus Verbindungen der Formel XXX ebenfalls wertvolle Substanzen erhalten werden, die über weitere Reaktionen zu Verbindungen der Formel XXIII führen.

**Schema 5:**

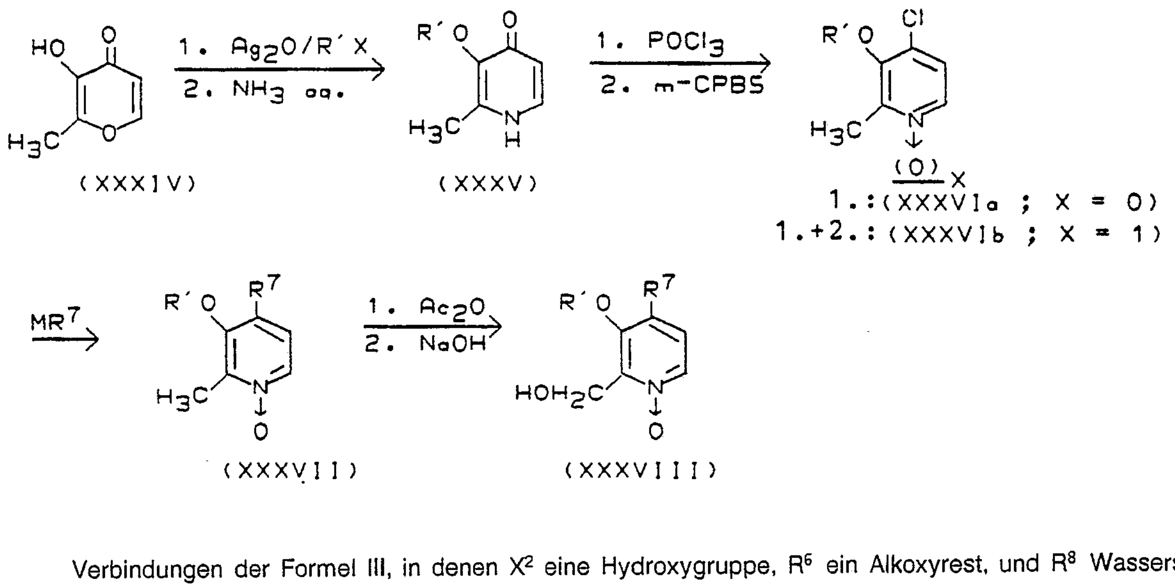


(XXXIV) (XXXV) 1.: (XXXVIa ; X = 0) 1.+2.: (XXXVIb ; X = 1) (XXXVII) (XXXVIII)

Verbindungen der Formel III, in denen $X^2$ eine Hydroxygruppe, $R^6$ ein Alkoxyrest, und $R^8$ Wasserstoff bedeutet, können nach Schema 5 hergestellt werden:

Maltol XXXIV alkyliert man mit einem Alkylhalogenid R´X in Gegenwart von Silberoxid zu 3-Alkoxypyran-4-onen, die mit wäßrigem Ammoniak zu 2-Methyl-3-alkoxy-4-pyridonen der Formel XXXV umgesetzt werden (J. Org. Chem. 29, 776 (1964)).

Substituierte 3-Benzyloxy-Derivate können in analoger Weise durch Alkylierung von Maltol mit den entsprechenden Benzylhalogeniden erhalten werden.

Durch analoge Umsetzungen mit 5-Hydroxy-2-methyl-4-pyranon, hergestellt aus Kojic Acid, werden die zu den Verbindungen der Formel XXXVIII isomeren Verbindungen mit $R^6$ = Wasserstoff und $R^8$ = Alkoxy erhalten.

Mit einem halogenierenden Agenz, z.B. $POCl_3$, werden die Verbindungen der Formel XXXV in 2-Methyl-3-alkoxy-4-chlor-pyridine überführt, aus denen mit einem Alkohol $R^7H$ in Gegenwart einer Base 4-Alkoxyderivate erhalten werden.

Vorteilhaft ist die Reaktion der analogen N-Oxide XXXVIb mit Alkoholaten zu Verbindungen der Formel XXXVII.

Verbindungen der Formel XXXV können in Gegenwart von Silberoxid auch direkt zu Verbindungen der Formel XXXIX alkyliert werden.

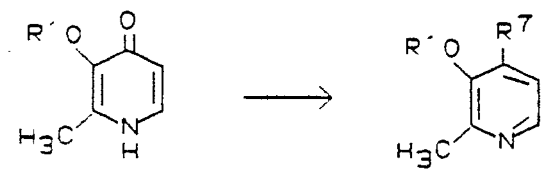

(XXXV) (XXXIX

$R^7$ = $(C_1-C_7)$-Alkoxy)

Verfahren zur Herstellung von 3,4-Dialkoxy-2-picolinen und 4,5-Dialkoxy-2-picolinen sind in der EP-A-166 287 und EP-A-208 452 beschrieben.

Verbindungen der Formel III, in denen $X^2$ eine Hydroxygruppe, einer der Substituenten $R^6$ und $R^8$ einen Alkoxysubstituenten oder einen halogenierten, vorzugsweise fluorierten Alkoxyrest $R_f$ bedeutet und der andere Wasserstoff, Halogen, Alkyl, Alkoxy oder ein halogenierter, vorzugsweise fluorierter Alkoxyrest $R_f$ sein kann, können nach Schema 6 hergestellt werden:

**Schema 6:**

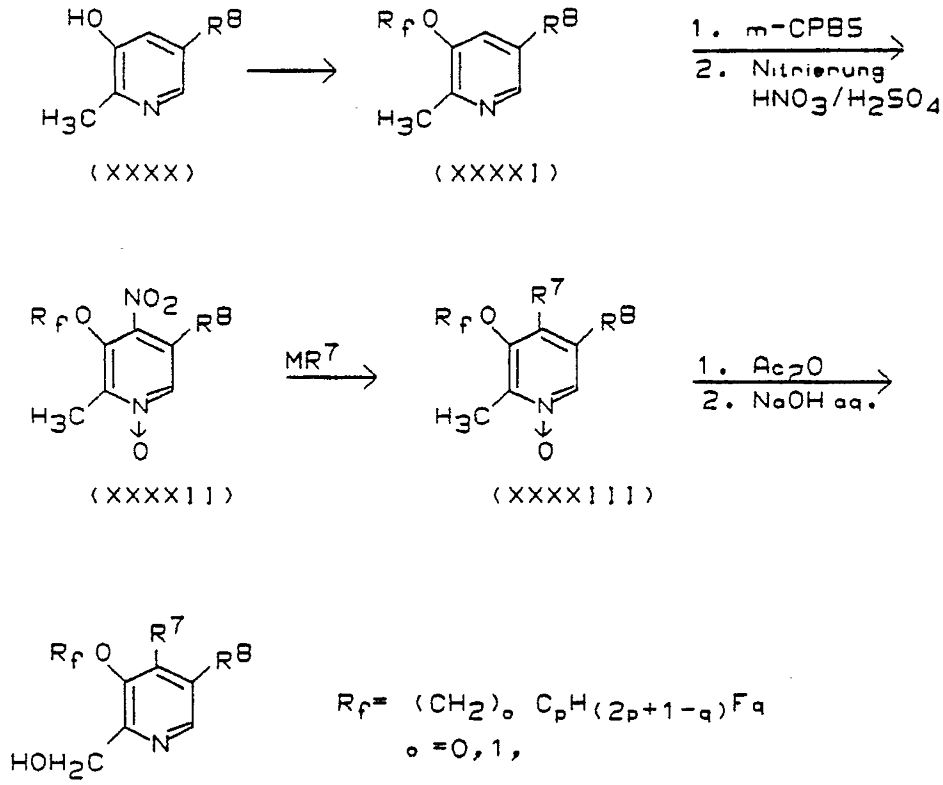


(XXXX) (XXXXI) (XXXXII) (XXXXIII) (XXXXIV)

$R_f = (CH_2)_o\ C_pH_{(2p+1-q)}F_q$

$o = 0, 1,$

Verbindungen der Formel XXXXI werden erhalten, indem 3-Hydroxy-2-picoline der Formel XXXX an entsprechende halogenierte Olefine (z.B. $CF_2 = CF_2$, $CF_2 = CClF$) addiert werden.

Weiterhin können aus Alkylhalogeniden und Base halogenierte Carbene, z.B. $CF_2$ aus $CHClF_2$, erzeugt werden, die durch Insertion in die OH-Bindung der 3-Hydroxy-2-picoline ebenfalls zu Verbindungen der Formel XXXXI führen (vgl. J. Org. Chem. 25, 2009 (1960)), 5-Trifluormethoxy-2-picolin wird erhalten, wie dies z.B. in J. Org. Chem. 29, 1 (1964) und J. Med. Chem. 13, 1124 (1970) beschrieben wird.

Die isomeren 5-Hydroxy-2-picoline können in analoger Weise umgesetzt werden.

Bei den in den obigen Schemata gezeigten Herstellungsverfahren besitzen die Substituenten und Variablen falls im Einzelfall nicht anders definiert, die einleitend gegebenen Definitionen.

Erfindungsgemäß werden außer den in den Ausführungsbeispielen beschriebenen Thienoimidazol-Derivaten beispielsweise auch die in der folgenden Tabelle I zusammengestellten Verbindungen der allgemeinen Formel I bzw. deren Salze erhalten.

Verwendete Abkürzungen:

Methyl (Me), Ethyl (Et), Propyl (Pr), Butyl (Bu), Hexyl (Hex), Acetyl (Ac), Phenyl (Ph), cyclo (c), iso (i).

**Tabelle**

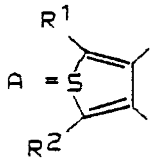

, T = SO

| $R^1$ | $R^2$ | $R^3$ | $R^4$ | $R^5$ | $R^6$ | $R^7$ | $R^8$ |
|---|---|---|---|---|---|---|---|
| H | H | H | H | H | H | OEt | Cl |
| H | H | H | H | H | H | OPr | Cl |
| H | H | H | H | H | H | OiPr | Cl |
| H | H | H | H | H | H | $OCH_2Ph$ | Cl |
| H | H | H | H | H | H | $O(CH_2)_2OMe$ | Cl |
| H | H | H | H | H | H | $OCH_2CF_3$ | Cl |
| H | H | H | H | H | H | $OCH_2CF_2CF_3$ | Cl |
| H | H | H | H | H | H | OEt | Br |
| H | H | H | H | H | H | OPr | Br |
| H | H | H | H | H | H | $OCH_2Ph$ | Br |
| H | H | H | H | H | H | $O(CH_2)_2OMe$ | Br |
| H | H | H | H | H | H | $OCH_2CF_3$ | Br |
| H | H | H | H | H | H | $OCH_2CF_2CF_3$ | Br |
| H | H | H | H | H | H | $OCH_2Ph$ | F |
| H | H | H | H | H | H | $O(CH_2)_2OMe$ | F |

Tabelle, Fortsetzung

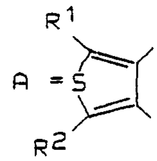

, T = SO

| $R^1$ | $R^2$ | $R^3$ | $R^4$ | $R^5$ | $R^6$ | $R^7$ | $R^8$ |
|---|---|---|---|---|---|---|---|
| H | H | H | H | H | H | $OCH_2CF_3$ | F |
| H | H | H | H | H | F | OMe | H |
| H | H | H | H | H | F | OEt | H |
| H | H | H | H | H | F | OPr | H |
| H | H | H | H | H | F | $OCH_2Ph$ | H |
| H | H | H | H | H | F | $O(CH_2)_2OMe$ | H |
| H | H | H | H | H | F | OMe | Me |
| H | H | H | H | H | F | OEt | Me |
| H | H | H | H | H | F | OPr | Me |
| H | H | H | H | H | F | OiPr | Me |
| H | H | H | H | H | F | $OCH_2Ph$ | Me |
| H | H | H | H | H | F | $O(CH_2)_2OMe$ | Me |
| H | H | H | H | H | F | $OCH_2CF_3$ | Me |
| H | H | H | H | H | Me | OEt | F |
| H | H | H | H | H | Me | OPr | F |
| H | H | H | H | H | Me | $OCH_2Ph$ | F |
| H | H | H | H | H | Me | $O(CH_2)_2OMe$ | F |
| H | H | H | H | H | Me | $OCH_2CF_3$ | F |
| H | H | H | H | H | H | OMe | F |
| H | H | H | H | H | H | OEt | F |
| H | H | H | H | H | H | OPr | F |
| H | H | H | H | H | H | OiPr | F |

Tabelle, Fortsetzung

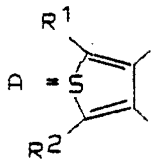

, T = SO

| $R^1$ | $R^2$ | $R^3$ | $R^4$ | $R^5$ | $R^6$ | $R^7$ | $R^8$ |
|---|---|---|---|---|---|---|---|
| H | H | H | H | H | Br | OEt | H |
| H | H | H | H | H | Br | OPr | H |
| H | H | H | H | H | Br | $OCH_2Ph$ | H |
| H | H | H | H | H | Br | $O(CH_2)_2OMe$ | H |
| H | H | H | H | H | Br | $OCH_2CF_3$ | H |
| H | H | H | H | H | $OCF_3$ | OMe | H |
| H | H | H | H | H | $OCF_3$ | OEt | H |
| H | H | H | H | H | $OCF_3$ | OPr | H |
| H | H | H | H | H | $OCF_3$ | $OCH_2Ph$ | H |
| H | H | H | H | H | $OCF_3$ | $O(CH_2)_2OMe$ | H |
| H | H | H | H | H | $OCF_3$ | $OCH_2CF_3$ | H |
| H | H | H | H | H | $OCF_2H$ | OMe | H |
| H | H | H | H | H | $OCF_2H$ | OEt | H |
| H | H | H | H | H | $OCF_2H$ | OPr | H |
| H | H | H | H | H | $OCF_2H$ | $O(CH_2)_2OMe$ | H |
| H | H | H | H | H | $OCF_2CF_2H$ | OMe | H |
| H | H | H | H | H | $OCF_2CF_2H$ | OMe | Me |
| H | H | H | H | H | $OCF_2CF_2H$ | OEt | Me |
| $CH_3$ | $CH_3$ | H | H | H | $OCF_2CF_2H$ | OMe | H |
| $CH_3$ | $CH_3$ | H | H | H | $OCF_2CF_2H$ | OMe | Me |
| $OCH_2CF_3$ | H | H | H | H | $OCF_2CF_2H$ | OMe | H |
| $OCH_2CF_3$ | H | H | H | H | $OCF_2CF_2H$ | OMe | Me |

Tabelle, Fortsetzung

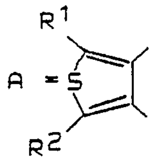 , T = SO

| $R^1$ | $R^2$ | $R^3$ | $R^4$ | $R^5$ | $R^6$ | $R^7$ | $R^8$ |
|---|---|---|---|---|---|---|---|
| $OCH_2CF_3$ | H | H | H | H | $OCF_2CF_2H$ | $OCH_2Ph$ | H |
| $OCH_2CF_3$ | H | H | H | H | H | OMe | $OCF_2CF_2H$ |
| $OCH_2CF_3$ | H | H | H | H | Me | OMe | $OCF_2CF_2H$ |
| $OCH_2CF_3CF_3$ | H | H | H | H | $OCF_2CF_2H$ | OMe | H |
| $OCH_2CF_2CF_3$ | H | H | H | H | $OCF_2CF_2H$ | OPr | H |
| $OCF_2CF_2H$ | H | H | H | H | $OCF_2CF_2H$ | OMe | H |
| $OCF_2CF_2H$ | H | H | H | H | $OCF_2CF_2H$ | OEt | H |
| $OCF_2CF_2H$ | H | H | H | H | $OCF_2CF_2H$ | $OCH_2Ph$ | H |
| $OCF_2CF_2H$ | H | H | H | H | OMe | OMe | $OCF_2CF_2H$ |
| H | H | H | H | H | H | OMe | $OCHF_2$ |
| H | H | H | H | H | H | OEt | $OCHF_2$ |
| H | H | H | H | H | H | $OCH_2Ph$ | $OCHF_2$ |
| H | H | H | H | H | H | $O(CH_2)_2OMe$ | $OCF_3$ |
| H | H | H | H | H | H | OMe | $OCF_3$ |
| H | H | H | H | H | H | OEt | $OCF_3$ |
| H | H | H | H | H | F | OMe | F |
| H | H | H | H | H | F | OEt | F |
| H | H | H | H | H | F | $OCH_2Ph$ | F |
| H | H | H | H | H | F | $O(CH_2)_2OMe$ | F |
| H | H | H | H | H | Cl | NPhMe | H |
| H | H | H | H | H | Cl | NPhMe | Me |

**Tabelle, Fortsetzung**

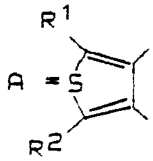

, T = SO

| $R^1$ | $R^2$ | $R^3$ | $R^4$ | $R^5$ | $R^6$ | $R^7$ | $R^8$ |
|---|---|---|---|---|---|---|---|
| H | H | H | H | H | H | NPhMe | Cl |
| H | H | H | H | H | Me | NPhMe | Cl |
| H | H | H | H | H | F | $-NMe_2$ | F |
| H | H | H | H | H | F | 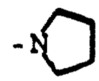 | F |
| H | H | H | H | H | F | 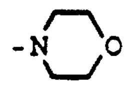 | F |
| H | H | H | H | H | Cl | $-NMe_2$ | Cl |
| H | H | H | H | H | Cl | 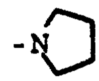 | Cl |
| H | H | H | H | H | Cl | 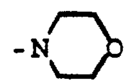 | Cl |
| $CH_3$ | $CH_3$ | H | H | H | $OCH_3$ | $OCH_2CF_3$ | H |
| $CH_3$ | $CH_3$ | H | H | H | $OCH_3$ | $OCH_2CF_2CF_2H$ | H |
| $CH_3$ | $CH_3$ | H | H | H | $OCH_3$ | $OCH_2CF_2CF_3$ | H |
| $CH_3$ | $CH_3$ | H | H | H | $OCH_3$ | $OCH_2CF_2CF_2CF_3$ | H |
| $CH_3$ | $CH_3$ | H | H | H | $OCH_3$ | $OCH_2CF_2CF_2CF_2CF_2H$ | H |
| H | H | H | H | H | H | $OCH_2CF_3$ | $OCH_3$ |
| H | H | H | H | H | H | $OCH_2CF_2CF_2H$ | $OCH_3$ |
| H | H | H | H | H | H | $OCH_2CF_2CF_3$ | $OCH_3$ |
| H | H | H | H | H | H | $OCH_2CF_2CF_2CF_3$ | $OCH_3$ |
| H | H | H | H | H | H | $OCH_2CF_2CF_2CF_2CF_2H$ | $OCH_3$ |
| $CH_3$ | $CH_3$ | H | H | H | H | $OCH_2CF_3$ | $OCH_3$ |
| $CH_3$ | $CH_3$ | H | H | H | H | $OCH_2CF_2CF_2H$ | $OCH_3$ |

Tabelle, Fortsetzung

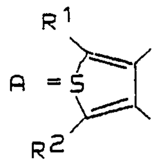

, T = SO

| $R^1$ | $R^2$ | $R^3$ | $R^4$ | $R^5$ | $R^6$ | $R^7$ | $R^8$ |
|---|---|---|---|---|---|---|---|
| $CH_3$ | $CH_3$ | H | H | H | H | $OCH_2CF_2CF_3$ | $OCH_3$ |
| $CH_3$ | $CH_3$ | H | H | H | H | $OCH_2CF_2CF_2CF_3$ | $OCH_3$ |
| $CH_3$ | $CH_3$ | H | H | H | H | $OCH_2CF_2CF_2CF_2CF_2H$ | $OCH_3$ |
| $OCH_2CF_3$ | H | H | H | H | $OCH_3$ | $OCH_2CF_3$ | H |
| $OCH_2CF_3$ | H | H | H | H | $OCH_3$ | $OCH_2CF_2CF_3$ | H |
| H | H | H | H | H | 4-Chlorbenzyloxy | $OCH_3$ | H |
| H | H | H | H | H | 4-Fluorbenzyloxy | $OCH_3$ | H |
| H | H | H | H | H | 2,4-Difluorbenzyloxy | $OCH_3$ | H |
| H | H | H | H | H | 3,5-Difluorbenzyloxy | $OCH_3$ | H |
| H | H | H | H | H | 4,6-Dichlorbenzyloxy | $OCH_3$ | H |
| H | H | H | H | H | 4,5-Dichlorbenzyloxy | $OCH_3$ | H |
| H | H | H | H | H | 4-Trifluormethylbenzyloxy | $OCH_3$ | H |
| H | H | H | H | H | 3,5-Bis-trifluormethylbenzyloxy | $OCH_3$ | H |
| H | H | H | H | H | 4-Fluorbenzyloxy | $OCH_2CF_3$ | H |
| H | H | H | H | H | 4-Chlorbenzyloxy | $OCH_2CF_3$ | H |
| H | H | H | H | H | 4-Trifluormethylbenzyloxy | $OCH_2CF_3$ | H |

**Tabelle, Fortsetzung**

A = 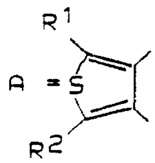 , T = SO

| $R^1$ | $R^2$ | $R^3$ | $R^4$ | $R^5$ | $R^6$ | $R^7$ | $R^8$ |
|---|---|---|---|---|---|---|---|
| H | H | H | H | H | 3,5-Bis-trifluormethyl-benzyloxy | $OCH_2CF_3$ | H |
| $CH_3$ | $CH_3$ | H | H | H | 4-Trifluor-methyl-benzyloxy | $OCH_2CF_3$ | H |
| H | H | H | H | H | H | $OCH_3$ | 4-Fluor-benzyl-oxy |
| H | H | H | H | H | H | $OCH_3$ | 4-Tri-fluor-methyl-benzyl-oxy |
| H | H | H | H | H | H | $OCH_2CF_3$ | 4-Fluor-benzyl-oxy |
| H | H | H | H | H | H | $OCH_2CF_3$ | 4-Tri-fluor-methyl-benzyl-oxy |

Die neuen Verbindungen der Formel I und ihre Salze besitzen wertvolle pharmakologische Eigenschaften.

Sie hemmen deutlich die Magensäuresekretion und weisen darüber hinaus eine ausgezeichnete Magen- und Darmschutzwirkung auf.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen, Bakterientoxine, Medikamente (z.B. Antiphlogistika und Antirheumatika), Chemikalien (z.B. Ethanol), Magensäure oder Streßsituationen verursacht werden können.

Aufgrund ihrer ausgezeichneten Eigenschaften sind die substituierten Thienoimidazole der Formel I und ihre pharmakologisch verträglichen Salze für den Einsatz in der Human- und Veterinärmedizin hervorragend geeignet, wobei sie insbesondere zur Behandlung und Prophylaxe von Krankheiten des Magens und Darms und solcher Krankheiten, die auf einer überhöhten Magensäuresekretion beruhen, verwendet werden.

Es wurde gefunden, daß auch die Colon-$H^+/K^+$-ATPase (vgl. Gustin, Goodman, J. Biol. Chem. 256 - [1981] 10651-10656) in vitro stark durch Verbindungen gehemmt wird, die beim Behandeln der erfindungsgemäßen Verbindungen der Formel I mit Säure (z.B. mit Natriumacetat/HCl-Puffer mit einem pH-Wert von etwa 4-5,5) entstehen. Solche Umwandlungsprodukte können sich auch in vivo bei der Passage der Verbindungen der Formel I durch den Magen-Darm-Trakt bilden. In welchem Umfang sie gebildet werden, hängt vom Substitutionsmuster und vom pH ab.

Der Colon-$H^+/K^+$-ATPase wird ein entscheidender Einfluß auf das Elektrolytgleichgewicht an der Darmschleimhaut zugeschrieben. Colon-$H^+/K^+$-ATPase-Hemmer, wie die oben genannten, können daher in dieses Gleichgewicht eingreifen und zur Behandlung von Krankheiten mit gestörtem Elektrolytgleichgewicht dienen.

Die Erfindung betrifft daher auch die Verwendung von Verbindungen der Formel I bzw. deren Säure-Umwandlungsprodukte bei der Behandlung von Durchfallerkrankungen. Beispiele solcher Krankheiten sind entzündliche Darmerkrankungen, wie Cholera, Paratyphus, Reisediarrhoe oder andere Formen der sekretorischen Diarrhoe aber auch andere Darmerkrankungen wie Morbus Crohn, Colitis ulcerosa und regionale Enteritis.

Die Erfindung betrifft weiterhin Umwandlungsprodukte, die beim Behandeln von Verbindungen der Formel I mit Säure gebildet werden.

Die Erfindung betrifft daher weiter die erfindungsgemäßen Verbindungen der Formel I zur Anwendung bei der Behandlung und Prophylaxe der vorstehend genannten Krankheiten.

Ebenso umfaßt die Erfindung die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere Verbindungen der allgemeinen Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoffe) entweder als solche, oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt vorteilhafterweise zwischen 0,1 und 96 % beträgt.

Welche Hilfsstoffe für die gewünschte Arzneimittelformulierungen geeignet sind, ist dem Fachmann aufgrund seines Wissens geläufig. Neben Lösemitteln, Gelbildern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können oral oder parenteral appliziert werden, wobei die orale Applikation bevorzugt ist.

Im allgemeinen hat es sich in der Humanmedizin als vorteilhaft erwiesen, den oder die Wirkstoffe bei oraler Gabe in einer Tagesdosis von etwa 0,01 bis etwa 20 mg/kg Körpergewicht, gegebenenfalls in Form mehrerer, vorzugsweise 1 bis 4 Einzelgaben zur Erzielung des gewünschten Ergebnisses zu verabreichen. Bei der parenteralen Applikation können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der obengenannten Krankheiten eingesetzt werden, so können die pharmazeutischen Zubereitungen auch einen oder mehrere pharmakologisch aktive Bestandteile anderer Arzneimittelgruppen, wie Antibiotika, beispielsweise Ofloxazin, Antacida, beispielsweise Aluminiumhydroxid, Magnesiumaluminat, Sucralfat, Bi-Salze, Tranquilizer, wie Benzodiazepine, beispielsweise Diazepam; Spasmolytika, wie z.B. Bietamiverin, Camylofin; Anticholinergica, wie z.B. Pirenzepin, Telenzepin, Oxyphencyclimin, Phencarbamid; Lokalanaesthetika, wie z.B. Tetracain, Procain; gegebenenfalls auch Gastrinantagonisten, Fermente, Vitamine oder Aminosäuren enthalten.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige alkoholische oder ölige Lösungen.

Als inerte Träger können z.B. Gummi arabicum, Magnesia, Magnesiumcarbonat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel

für die neuen aktiven Verbindungen und die entsprechenden physiologisch verträglichen Salze kommen z.B. in Frage: Wasser, physiologische Kochsalzlösungen oder Alkohole, z.B. Ethanol, Propanol oder Glycerin, daneben auch Zuckerlösungen wie Glucose- oder Mannitlösungen, oder auch eine Mischung aus den verschiedenen genannten Lösungsmitteln.

Die nachfolgenden Beispiele sollen die erfindungsgemäßen Verfahrensweisen erläutern, ohne die Erfindung auf die hier stellvertretend genannten Substanzen zu beschränken.

Die angegebenen Schmelz- und Zersetzungspunkte sind nicht korrigiert oder standardisiert.

**5-Chlor-2-picolin**

**a) 5-Chlor-2-picolin-N-oxid**

13,0 g 5-Chlor-2-picolin werden in 250 ml Methylenchlorid unter Rühren bei Raumtemperatur portionsweise mit 21,3 g m-Chlorperbenzoesäure (m-CPBS) versetzt. Nach 2 Stunden wird 2mal mit je 200 ml gesättigter wäßriger $NaHCO_3$-Lösung und anschließend dreimal mit je 200 ml gesättigter wäßriger $NaHSO_3$-Lösung ausgeschüttelt. Die Bicarbonatlösung wird dreimal mit je 100 ml Methylenchlorid extrahiert, die vereinigten organischen Phasen getrocknet und vom Lösungsmittel befreit. Nach dem Verreiben mit Petrolether erhält man die kristalline Titelverbindung, Fp. 73-76° C.

**b) 4-Nitro-5-chlor-2-picolin-N-oxid**

Unter Kühlung werden 12 g 5-Chlor-2-picolin-N-oxid portionsweise in eine Mischung aus 40 ml konzentrierter Schwefelsäure und 40 ml rauchender Salpetersäure eingetragen. Anschließend wird 1 Stunde auf 35 bis 40° C und 2 Stunden auf 65 bis 70° C erwärmt. Nach dem Abkühlen wird mit 250 g Eis gerührt, mit 10 N Natronlauge auf pH 10 bis 11 gebracht und mit Essigester extrahiert. Nach dem Befreien vom Lösungsmittel wird der Rückstand mit Diisopropylether behandelt, Fp. 115-117° C.

**c) 4-Methoxy-5-chlor-2-picolin-N-oxid**

Zu einer Natriummethylatlösung, hergestellt aus 1,5 g Natrium und 150 ml Methanol, gibt man bei -10° C 3,5 g 4-Nitro-5-chlor-2-picolin-N-oxid in 50 ml wasserfreiem Methanol. Man läßt langsam auf Raumtemperatur erwärmen und erhitzt sodann 1 Stunde am Rückfluß. Nun destilliert man das Lösungsmittel unter vermindertem Druck ab, versetzt den Rückstand mit Wasser, extrahiert mit Dichlormethan und vertreibt das Lösungsmittel. Farblose Kristalle aus Diisopropylether, Fp. 137-139° C.

**d) 5-Chlor-2-hydroxymethyl-4-methoxypyridin**

8,5 g 3-Chlor-4-methoxy-2-picolin-N-oxid werden in 16 ml Eisessig gelöst und unter Rühren bei 80° C mit 24 ml Acetanhydrid versetzt. Man erhitzt 1,5 Stunden auf 110 bis 120° C, läßt sodann auf 80° C abkühlen und tropft 40 ml Methanol zu. Anschließend wird unter vermindertem Druck eingeengt, dann der Rückstand mit 35 ml Wasser, 13,6 g Ätznatron in kleinen Portionen und mit 15 ml Dioxan versetzt und diese Mischung 2 Stunden zum Rückfluß erhitzt. Nach dem Abkühlen extrahiert man mit Dichlormethan, vertreibt das Lösungsmittel und bringt den Rückstand mit Diisopropylether zur Kristallisation. Feststoff, Fp. 78-80° C.

**e) 5-Chlor-2-chlormethyl-4-methoxypyridin-hydrochlorid**

Zu einer Mischung aus 4,5 g 5-Chlor-2-hydroxymethyl-4-methoxypyridin und 100 ml Dichlormethan tropft man bei 0° C eine Lösung aus 8,0 ml Thionylchlorid in 20 ml Dichlormethan und rührt sodann 2 Stunden bei Raumtemperatur. Man vertreibt das Lösungsmittel und bringt den Rückstand mit Diethylether zur Kristallisation. Farblose Kristalle, Fp. 136-137° C.

**f) 2-(4-Methoxy-5-chlor-2-picolylmercapto)-1H-thieno[3,4-d]imidazol-dihydrochlorid**

1,56 g 2-Mercapto-thieno[3,4-d]imidazol und 2,3 g 4-Methoxy-5-chlor-2-picolylchlorid-hydrochlorid werden in 100 ml Ethanol etwa 1 Stunde auf 60 °C erwärmt, anschließend 1,5 Stunden zum Rückfluß erhitzt. Nach dem Abfiltrieren schlämmt man die kristalline Substanz in Aceton auf, rührt 1 Stunde bei Raumtemperatur, saugt die Kristalle ab und trocknet an der Luft, Fp. > 260 °C.

Das Filtrat wird mit Tierkohle behandelt, im Vakuum eingeengt und mit Aceton zur Kristallisation gebracht, Fp. > 260 °C.

**g) 2-(4-Methoxy-5-chlor-2-picolylmercapto)-1H-thieno[3,4,-d]imidazol**

1,4 g der Titelverbindung aus Beispiel 1 f) werden nach Suspendieren in 70 ml Methanol bei Raumtemperatur unter Rühren mit 2 ml Triethylamin versetzt. Man rührt die so erhaltene Lösung etwa 1 Stunde bei Raumtemperatur, schlämmt mit Aktivkohle auf, filtriert und engt im Vakuum ein. Der Rückstand wird mit Wasser versetzt und mit Methylenchlorid extrahiert. Nach Abdampfen des Lösungsmittels bringt man den Rückstand mit Diisopropylether zur Kristallisation, Fp. 135-137 °C.

**h) 2-(4-Methoxy-5-chlor-2-picolylsulfinyl)-1H-thieno[3,4,-d]imidazol**

Zu 0.44 g der Titelverbindung aus Beispiel 1 g) werden im Zweiphasengemisch aus 70 ml Methylenchlorid und 40 ml wäßriger $KH_2PO_4$/$Na_2HPO_4$-Pufferlösung (pH = 7,5) unter Rühren bei 0 bis 5 °C 0,308 g m-Chlorperbenzoesäure (85 %) in 10 ml Methylenchlorid hinzugetropft. Nach etwa 30 Minuten wird die organische Phase eingeengt und der Rückstand mit Dichlormethan/Ethylacetat an Kieselgel chromatographiert. Aus den entsprechenden Fraktionen wird das Produkt mit Ethylacetat zur Kristallisation gebracht, Fp. 159 °C [Zers.], oder aus Ethanol umkristallisiert.

**Beispiel 2**

**a) 3-Brom-2-picolin-N-oxid und 5-Brom-2-picolin-N-oxid**

Analog Beispiel 1 a) werden die Titelverbindungen aus 43 g Gemisch, bestehend aus 3-Brom-2-picolin und 5-Brom-2-picolin mit 60 g m-Chlorperbenzoesäure (85 %) erhalten. Das Gemisch der Titelverbindungen wird mit Diisopropylether zur Kristallisation gebracht, Fp. 65-80 °C (unscharf).

**b) 3-Brom-4-nitro-2-picolin-N-oxid und 5-Brom-4-nitro-2-picolin-N-oxid**

Analog Beispiel 1 b) werden 26,3 g der Titelverbindungen aus Beispiel 2 a) in 50 ml $H_2SO_4$ / 50 ml $HNO_3$ 7 Stunden bei 60 bis 65 °C gerührt.

Aus dem Eindampfrückstand kristallisiert mit einem Gemisch aus Essigsäureethylester und Diisopropylether (1 : 1) bevorzugt das 5-Isomere, Fp. 133-135 °C. Bei der chromatographischen Trennung der Mutterlauge mit Toluol / Ethylacetat (1 : 1) an Kieselgel wird zunächst weiteres 5-Brom-4-nitro-2-picolin-N-oxid eluiert, gefolgt von 3-Brom-4-nitro-2-picolin-N-oxid, Fp. 111-113 °C (aus Petrolether).

**Beispiel 3**

a) **4-Methoxy-5-brom-2-picolin-N-oxid**

Analog Beispiel 1 c) wird die Titelverbindung aus 8,2 g 4-Nitro-5-brom-2-picolin-N-oxid und 0,87 g Na in Methanol erhalten. Nach Abdampfen des Dichlormethans wird der Rückstand mit Diisopropylether zur Kristallisation gebracht, Fp. 146-148 °C.

b) **4-Methoxy-5-brom-2-hydroxymethylpyridin**

Analog Beispiel 1 d) werden 6,6 g 4-Methoxy-5-brom-2-picolin-N-Oxid mit 7 ml Eisessig, 15 ml Acetanhydrid, 20 ml Methanol, 20 ml Wasser, 8 g NaOH und 20 ml Dioxan umgesetzt. Das Rohprodukt wird mit Diisopropylether zur Kristallisation gebracht, Fp. 80-82 °C.

c) **4-Methoxy-5-brom-2-chlormethylpyridin-hydrochlorid**

Analog Beispiel 1 e) wird die Titelverbindung aus 4,3 g der Titelverbindung von Beispiel 3b) und 5,0 ml Thionylchlorid erhalten, Fp. > 300 °C (aus Diisopropylether).

d) **2-(4-Methoxy-5-brom-2-picolylmercapto)-1H-thieno[3,4-d]imidazol-dihydrochlorid**

1,2 g 2-Mercapto-thieno[3,4-d]imidazol werden in 50 ml Ethanol suspendiert, mit 2,0 g 4-Methoxy-5-brom-2-chlormethylpyridin-hydrochlorid versetzt und 1 Stunde bei 60 °C gerührt. Man befreit vom Lösungsmittel und bringt den Rückstand mit Diethylether zur Kristallisation, Fp. > 300 °C.

e) **2-(4-Methoxy-5-brom-2-picolylmercapto)-1H-thieno[3,4-d]imidazol**

2,15 g der Titelverbindung aus Beispiel 3 d) werden in 50 ml Methanol mit 7 ml Triethylamin versetzt. Analog Beispiel 1 g) kristallisiert das Produkt bei Versetzen mit Wasser, Fp. 165 °C [Zers.].

f) **2-(4-Methoxy-5-brom-2-picolylsulfinyl)-1H-thieno[3,4-d]imidazol**

Die Titelverbindung wird analog Beispiel 1 h) aus 1,1 g der Titelverbindung von Beispiel 3 e) und 0,725 g m-Chlorbenzoesäure erhalten. Beim Behandeln des Rückstands kristallisiert die Titelverbindung aus wenig Diethylether/Ethylacetat (1 : 1), Fp. 140 °C [Zers.]

Weiteres Produkt erhält man aus der sich dunkel verfärbenden Mutterlauge durch Eindampfen und sofortiges Behandeln mit wenig Ethylacetat.

**Beispiel 4**

a) **3-Brom-4-methoxy-2-picolin-N-oxid**

Analog den Beispielen 1 c) und 3 a) wird die Titelverbindung aus 4,6 g 3-Brom-4-nitro-2-picolin-N-oxid in 40 ml Methanol mit einer Natriummethylat-Lösung, hergestellt aus 0,5 g Natrium und 20 ml Methanol, erhalten, Fp. 80-82 °C (aus Diisopropylether).

b) **3-Brom-4-methoxy-2-hydroxymethylpyridin**

3,8 g der Titelverbindung aus Beispiel 4a) werden 1 Stunde bei 80-85 °C mit 30 ml Acetanhydrid gerührt. Analog den Beispielen 1 d)/3 b) wird mit 25 ml Methanol, nach dem Einengen mit 30 ml 5 N Natronlauge und 10 ml Dioxan versetzt. Das Produkt kristallisiert aus Diisopropylether, Fp. 109-111 °C.

c) 3-Brom-4-methoxy-2-chlormethylpyridin-hydrochlorid

Analog den Beispielen 1 e) und 3 c) werden 1,9 g der Titelverbindung von Beispiel 4 b) mit 2,5 ml Thionylchlorid umgesetzt. Das Produkt wird mit Diisopropylether zur Kristallisation gebracht, Fp. 142-144° C.

d) 2-(3-Brom-4-methoxy-2-picolylmercapto)-1H-thieno[3,4-d]imidazol-dihydrochlorid

1,3 g 2-Mercapto-thieno[3,4-d]imidazol werden in 50 ml Ethanol unter Rühren mit 2,3 g 3-Brom-4-methoxy-2-chlormethyl-pyridin-hydrochlorid versetzt und 30 Minuten bei 60° gehalten. Das Produkt wird abgesaugt und im Vakuum getrocknet, Fp. > 300° C.

e) 2-(3-Brom-4-methoxy-2-picolylmercapto)-1H-thieno[3,4-d]imidazol

Analog Beispiel 1 g) werden 2,4 g des Dihydrochlorids aus Beispiel 4 d) in 50 ml Methanol mit 2,9 ml Triethylamin versetzt. Nach dem Einengen und Versetzen mit wenig Wasser kristallisiert der Rückstand, Fp. 168° C [Zers.].

f) 2-(3-Brom-4-methoxy-2-picolylsulfinyl)-1H-thieno[3,4-d]imidazol

Analog Beispiel 1 h) werden zu 0,72 g der Titelverbindung aus Beispiel 4 e) in 120 ml Dichlormethan und 50 ml wäßriger $KH_2PO_4/Na_2HPO_4$-Pufferlösung (pH = 7,5) 0,44 g m-Chlorperbenzoesäure (85 %) in 15 ml Dichlormethan hinzugetropft. Nach dem Einengen der organischen Phase bringt man den Rückstand mit Ethylacetat zur Kristallisation, Fp. 160° C (Polymerisation?).

**Beispiel 5**

a) 2-Methyl-3-methoxy-4-chlor-pyridin-N-oxid

11,2 g 3-Methoxy-2-methyl-4(1H)-pyridon werden in 100 ml Phosphoroxychlorid 10 Stunden rückfließend erhitzt. Anschließend engt man ein, versetzt 2mal mit je 30 ml Toluol, engt wiederum ein, nimmt den Rückstand in 150 ml Wasser auf, bringt mit $K_2CO_3$ auf pH 11, extrahiert mit Dichlormethan, wäscht die organische Phase mit Wasser, trocknet und befreit vom Lösungsmittel.

Aus dem hellbraunen Öl (9 g) wird mit m-Chlorperbenzoesäure in Dichlormethan unter Standardbedingungen die Titelverbindung erhalten, Fp. 88-89° C (aus Petrolether).

b) 2-Methyl-3,4-dimethoxy-pyridin-N-oxid

Zu 8 g der Titelverbindung aus Beispiel 5 a) in 100 ml wasserfreiem Methanol tropft man eine aus 1,14 g Natrium und 50 ml Methanol hergestellte Natriummethylatlösung und erhitzt 20 Stunden zum Sieden, engt ein, versetzt mit Wasser, extrahiert mit Dichlormethan und erhält nach Einengen die Titelverbindung, Fp. 111-113° C.

c) 3,4-Dimethoxy-2-hydroxymethyl-pyridin

5,8 g des vorstehend beschriebenen Produkts werden analog Beispiel 1 d) in 10 ml Eisessig, 15 ml Acetanhydrid, 20 ml Methanol, 20 ml Wasser, 8 g NaOH 20 ml Dioxan umgesetzt, Fp. 86-88° C (aus Diisopropylether).

**d)3,4-Dimethoxy-2-chlormethyl-pyridin-hydrochlorid**

3,4 g des vorstehenden Produkts werden analog Beispiel 1 e) mit 5 ml Thionylchlorid umgesetzt. Fp. 150-151 °C [Zers.] (aus Diethylether).

**e) 2-(3,4-Dimethoxy-2-picolylmercapto)-1H-thieno[3,4-d]imidazol**

Analog Beispiel 1 f) werden 1,56 g 2-Mercaptothieno[3,4-d]imidazol in 70 ml Ethanol mit 2,3 g 3,4-Dimethoxy-2-chlormethyl-pyridin-hydrochlorid umgesetzt. Fp. 148-150 °C [Zers.]
Analog Beispiel 1 g) wird anschließend in 60 ml Methanol mit 2,5 ml Triethylamin behandelt. Fp.141-142 °C [Zers.]

**f) 2-(3,4-Dimethoxy-2-picolylsulfinyl)-1H-thieno[3,4]imidazol**

Analog Beispiel 1 h) werden 0,75 g der Titelverbindung aus Beispiel 5 e) in 100 ml Dichlormethan, 50 ml $Na_2HPO_4/KH_2PO_4$-Pufferlösung (pH = 7,5), mit 0,55 g m-Chlorperbenzoesäure oxidiert. Nach dem Einengen wird mit Diethylether zur Kristallisation gebracht, Fp. 139-140 °C.

**Beispiel 6**

**a) 2-Brommethyl-3-fluorpyridin**

1,11 g 3-Fluor-2-picolin werden in 50 ml $CCl_4$ gelöst mit 3,6 g N-Bromsuccinimid und 0,1 g Azobisisobutyronitril versetzt und 2 Stunden unter Rückflußkochen gerührt. Nach dem Abkühlen und Filtration wird das Lösungsmittel im Vakuum entfernt. Das Rohprodukt zeigt im Massenspektrum den erwarteten Molpeak von 189 m/e.

**b) 2-(3-Fluor-2-picolylmercapto-1H-thieno[3,4-d]imidazol**

1,6 g 2-Mercapto-thieno[3,4-d]imidazol und 1,9 g 2-Brommethyl-3-fluorpyridin werden mit 2,8 g gepulvertem, wasserfreiem $K_2CO_3$ in ca. 100 ml Aceton während 4 Stunden ohne Kühlung gerührt. Nach Filtration und Entfernen des Lösungsmittels im Vakuum reinigt man das Rohprodukt durch Säulenchromatographie an Kieselgel mit einem Laufmittelgemisch aus Cyclohexan/Essigester. Die Produktfraktion zeigt einen $R_f$ von ca. 0,15.

**c) 2-(3-Fluor-2-picolylsulfinyl-1H-thieno[3,4-d]imidazol**

530 mg der Verbindung aus der vorhergehenden Stufe werden in 50 ml $CH_2Cl_2$ gelöst und bei Raumtemperatur mit 444 mg m-Chlorperbenzoesäure 4 Stunden gerührt. Die Lösung wird nacheinander mit $NaHSO_3$-Lösung, $NaHCO_3$-Lösung und $H_2O$ gewaschen und mit $Na_2SO_4$ getrocknet. Das nach dem Entfernen des Lösungsmittels erhaltene Rohprodukt wird der Säulenchromatographie unterworfen ($SiO_2$; $CH_2Cl_2$: Aceton = 7/3). Die Produktfraktionen werden eingeengt und mit Diisopropylether verrührt und abfiltriert. Fp. 145 °C; MS ergibt einen Molpeak von 282 m/e; $(M+H)^+$.

Die Verbindungen der Formel I der nachstehenden Beispiele 7 bis 22 werden analog der in Schemata 1 bis 6 angegebenen Verfahrensweisen erhalten:

| Bsp. | T | $R^3$ | $R^6$ | $R^7$ | $R^8$ | Fp. |
|---|---|---|---|---|---|---|
| 7 | S | H | $CH_3$ | $OCH_3$ | F | 170-173 °C |
| 8 | SO | H | $CH_3$ | $OCH_3$ | F | ab 145 °C [Zers.] |
| 9 | S | H | H | H | $OCHF_2$ | (MS: $M^+ = 313$) |
| 10 | SO | H | H | H | $OCHF_2$ | 116 °C [Zers.] MS(DCI): $M^+ + 1 = 329$ |
| 11 | S | H | H | H | Cl | 160 °C [Zers.] |
| 12 | SO | H | H | H | Cl | 157 °C [Zers.] |
| 13 | S | H | $OCH_3$ | H | H | 180 °C [Zers.] |
| 14 | SO | H | $OCH_3$ | H | H | 85-90 °C [Zers.] |
| 15 | S | H | H | H | $OCH_3$ | 112-114 °C |
| 16 | SO | H | H | H | $OCH_3$ | 152-154 °C [Zers.] |
| 17 | S | H | $OCHF_2$ | H | H | 103 °C [Zers.];$M^+ = 313$ |
| Bsp. | T | $R^3$ | $R^6$ | $R^7$ | $R^8$ | Fp. |
| 18 | SO | H | $OCHF_2$ | H | H | 78 °C [Zers.];$M + H^+ = 330$ |
| 19 | S | H | $OCH_2CF_3$ | $OCH_2CF_3$ | H | 145 °C [Zers.];$M^+ = 443$ |
| 20 | SO | H | $OCH_2CF_3$ | $OCH_2CF_3$ | H | 154 °C [Zers.];$M + H^+ = 460$ |
| 21 | S | H | $OCF_2CF_2H$ | $OCH_3$ | H | Öl ;$M + H^+ = 394$ |
| 22 | SO | H | $OCF_2CF_2H$ | $OCH_3$ | H | 138 °C [Zers.];$M + H^+ = 410$ |
| $R^1$, $R^2$, $R^4$ und $R^5$ sind jeweils Wasserstoff | | | | | | |

**Beispiel 23**

**a) 2-Methyl-3-methoxy-4-(2,2,2-trifluorethoxy)pyridin-N-oxid**

Zu 20 ml Trifluorethanol gibt man bei -20 °C unter Rühren und Stickstoffatmosphäre portionsweise 6,7 g Kalium-tert.Butylat. Nach Erwärmung auf 0 °C werden portionsweise 5,2 g 2-Methyl-3-methoxy-4-chlor-pyridin-N-oxid (Titelverbindung aus Beispiel 5 a) hinzugegeben. Man erwärmt 3 Stunden unter Rückfluß, läßt auf Raumtemperatur abkühlen, gibt weitere 3,45 g Kalium-tert.Butylat zu und erwärmt 2 Stunden unter Rückfluß. Nach dem Abkühlen gibt man 40 ml Wasser zum Reaktionsgemisch, extrahiert mit Dichlormethan, trocknet über $MgSO_4$ und befreit im Vakuum vom Lösungsmittel. Das erhaltene ölige Rohprodukt wird weiter umgesetzt.

**b) 3-Methoxy-4-(2,2,2-trifluorethoxy)-2-hydroxy-methyl-pyridin**

8 g der Titelverbindung aus Beispiel 23 a) werden in 16 ml Eisessig gelöst und unter Rühren bei 80 °C mit 24 ml Acetanhydrid versetzt. Man erhitzt 2 Stunden auf 110 °C, kühlt sodann auf 80 °C ab und tropft 40 ml Methanol zur Reaktionsmischung. Anschließend wird im Vakumm eingeengt, der ölige Rückstand zu 75 ml 2 N methanolischer NaOH hinzugegeben und 30 Minuten gerührt. Nach Behandeln mit Aktivkohle und Filtration wird im Vakuum eingeengt, der Rückstand mit 50 ml Wasser versetzt, mit Dichlormethan extrahiert, getrocknet ($MgSO_4$), eingeengt und der Rückstand mit Diisopropylether behandelt. Farblose Kristalle, Fp. 107-108 °C.

**c) 3-Methoxy-4-(2,2,2-trifluorethoxy)-2-chlormethyl-pyridin-hydrochlorid**

Zu einer Mischung aus 3,9 g des vorstehenden Produkts und 100 ml Dichlormethan tropft man bei 0 °C eine Lösung aus 6,0 ml Thionylchlorid in 15 ml Dichlormethan und rührt sodann 2 Stunden bei Raumtemperatur. Man vertreibt das Lösungsmittel und bringt den Rückstand mit Diisopropylether zur Kristallisation. Farblose Kristalle, Fp. 166-168 °C.

**d) 2-(3-Methoxy-4-(2,2,2-trifluorethoxy)-2-picolylmercapto)-1H-thieno[3,4-d]imidazol-dihydrochlorid**

1,0 g 2-Mercapto-thieno[3,4-d]imidazol und 1,9 g des vorstehenden Produkts werden in 60 ml Ethanol etwa 1 Stunde auf 60 °C erwärmt, anschließend 1,5 Stunden zum Rückfluß erhitzt. Nach Behandlung mit Aktivkohle, Abfiltrieren, Einengen bringt man den Rückstand mit Aceton zur Kristallisation. Fp. ab 188 °C (Zers.).

**e) 2-(3-Methoxy-4-(2,2,2-trifluorethoxy)-2-picolylmercapto)-1H-thieno[3,4-d]imidazol**

2,1 g der Titelverbindung aus Beispiel 23 d) werden nach dem Suspendieren in 80 ml Methanol bei Raumtemperatur unter Rühren mit 4 ml Triethylamin versetzt. Man rührt die so erhaltene Lösung etwa 1 Stunde bei Raumtemperatur, schlämmt mit Aktivkohle auf, filtriert und engt im Vakuum ein. Beim Versetzen mit Wasser kristallisiert das farblose Produkt. Fp. 147-148 °C.

**f) 2-(3-Methoxy-4-(2,2,2-trifluorethoxy)-2-picolylsulfinly)-1H-thieno[3,4-d]imidazol**

Zu 0,75 g der Titelverbindung aus Beispiel 23 e) werden im Zweiphasengemisch aus 80 ml Methylenchlorid und 40 ml wäßriger $KH_2PO_4/Na_2HPO_4$-Pufferlösung (pH = 7,5) unter Rühren bei 0 bis 5 °C 0,42 g m-Chlorperbenzoesäure (85 %) in 10 ml Methylenchlorid hinzugetropft. Nach etwa 30 Minuten wird die organische Phase abgetrennt, getrocknet, eingeengt und der Rückstand mit Diethylether zur Kristallisation gebracht. Fp. 135-137 °C.

Die nachstehenden Zwischenprodukte der Formeln XXXXVI-XXXXVIII können beispielsweise analog Schema 5 hergestellt werden:

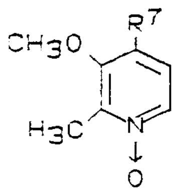

XXXXVI

| Bsp.Nr. | $R^7$ | Fp. |
|---|---|---|
| 24 | $OCH_2CF_2CF_2H$ | 40-42 °C |
| 25 | $OCH_2CF_2CF_3$ | 138 °C |
| 26 | $OCH_2CF_2CF_2CF_3$ | 128-130 °C |
| 27 | $OCH_2CF_2CF_2CF_2CF_2H$ | Öl* |

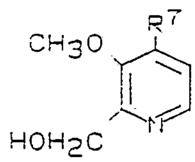

XXXXVII

| Bsp.Nr. | $R^7$ | Fp. |
|---|---|---|
| 28 | $OCH_2CF_2CF_2H$ | 45-47 °C |
| 29 | $OCH_2CF_2CF_3$ | Öl* |
| 30 | $OCH_2CF_2CF_2CF_3$ | 68-70 °C |
| 31 | $OCH_2CF_2CF_2CF_2CF_2H$ | Öl* |

* wurde direkt umgesetzt

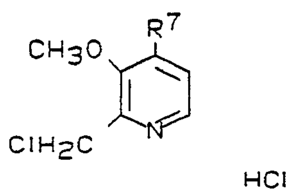

XXXXVIII

| Bsp.Nr. | $R^7$ | Fp. |
|---|---|---|
| 32 | $OCH_2CF_2CF_2H$ | 133 °C Zers. |
| 33 | $OCH_2CF_2CF_3$ | 155 °C |
| 34 | $OCH_2CF_2CF_2CF_3$ | 161-163 °C |
| 35 | $OCH_2CF_2CF_2CF_2CF_2H$ | 132 °C |

Die nachstehenden Verbindungen der Formel I werden, analog den Beispielen 5 und 23 erhalten:

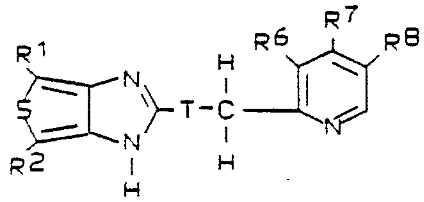

| Bsp.Nr. | T | $R^1$ | $R^2$ | $R^6$ | $R^7$ | $R^8$ | Fp. (°C) |
|---|---|---|---|---|---|---|---|
| 36 | S | H | H | $OCH_3$ | $OCH_2CF_2CF_2H$ | H | 127-129 |
| 37 | S | H | H | $OCH_3$ | $OCH_2CF_2CF_3$ | H | 142 |
| 38 | S | H | H | $OCH_3$ | $OCH_2CF_2CF_2CF_3$ | H | 133-135 |
| 39 | S | H | H | $OCH_3$ | $OCH_2CF_2CF_2CF_2CF_2H$ | H | 110 |
| 40 | SO | H | H | $OCH_3$ | $OCH_2CF_2CF_2H$ | H | 133-135 |
| 41 | SO | H | H | $OCH_3$ | $OCH_2CF_2CF_3$ | H | 137 Zers. |
| 42 | SO | H | H | $OCH_3$ | $OCH_2CF_2CF_2CF_3$ | H | 113-115 |
| 43 | SO | H | H | $OCH_3$ | $OCH_2CF_2CF_2CF_2CF_2H$ | H | 103 Zers. |
| 44 | S | H | Phenyl | H | H | H | 153-157 |
| 45 | S | H | Phenyl | H | $OCH_3$ | H | 182-185 |

| Bsp. Nr. | T | $R^1$ | $R^2$ | $R^6$ | $R^7$ | $R^8$ | Fp. (°C) |
|---|---|---|---|---|---|---|---|
| 46 | S | H | Phenyl | $CH_3$ | $OCH_3$ | $CH_3$ | 192-195 |
| 47 | S | H | 4-Methoxy-phenyl | H | H | H | 141-143 |
| 48 | S | H | " | H | $OCH_3$ | H | 170-173 |
| 49 | S | H | " | $CH_3$ | $OCH_3$ | $CH_3$ | 151-154 |
| 50 | S | H | Phenyl | H | $OCH_2CF_2CF_2CF_3$ | H | 216 |
| 51 | S | H | 4-Methoxy-phenyl | H | $OCH_2CF_2CF_2CF_3$ | H | 203 |
| 52 | S | $OCH_3$ | $CO_2CH_3$ | H | H | H | 106-110 |
| 53 | S | H | $CON(C_2H_5)_2$ | H | H | H | 102-104 · HCl |
| 54 | SO | H | Phenyl | H | H | H | 137-141 |
| 55 | SO | H | " | H | $OCH_3$ | H | 130-135 |
| 56 | SO | H | " | $CH_3$ | $OCH_3$ | $CH_3$ | 160-165 |
| 57 | SO | H | 4-Methoxy-phenyl | H | H | H | 164-167 Zers. |
| 58 | SO | H | " | H | $OCH_3$ | H | 162-165 |
| 59 | SO | H | " | $CH_3$ | $OCH_3$ | $CH_3$ | 162-165 Zers. |
| 60 | SO | H | Phenyl | H | $OCH_2CF_2CF_2CF_3$ | H | 80 |
| 61 | SO | H | 4-Methoxy-phenyl | H | $OCH_2CF_2CF_2CF_3$ | H | 79 |

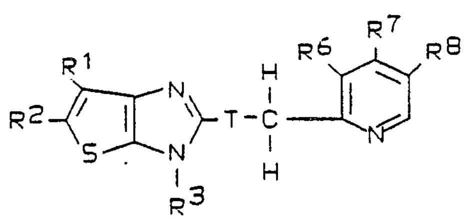

| Bsp. Nr. | T | $R^1$ | $R^2$ | $R^6$ | $R^7$ | $R^8$ | Fp. (°C) |
|---|---|---|---|---|---|---|---|
| 62 | S | $-CH_2SCH_2-$ | | H | H | H | 186-188 |
| 63 | S | $CO_2CH_3$ | $CO_2CH_3$ | H | $OCH_3$ | H | 156-159 |
| 64 | SO | $CO_2CH_3$ | $CO_2CH_3$ | H | $OCH_3$ | H | 140 Zers. |
| 65 | SO | $CO_2CH_3$ | $CO_2CH_3$ | H | H | H | 146-149 |

**Ansprüche**

1. Verbindung der Formel I

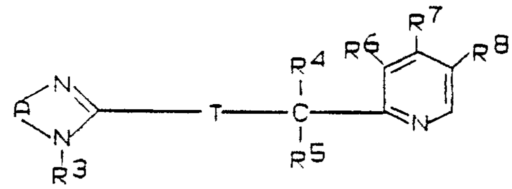

(I)

in welcher

A für a) 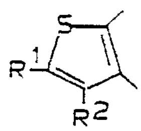 , b) 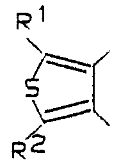 oder c) 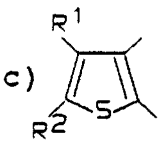 steht,

T -S-, -SO- oder $-SO_2-$ bedeutet,
$R^1$ und $R^2$ gleich oder verschieden sind und Wasserstoff, Halogen, Cyano, Nitro, Trifluormethyl, $(C_1-C_6)$-Alkyl, $(C_1-C_6)$-Hydroxyalkyl, $(C_1-C_6)$-Alkoxy, $-O-[CH_2]_o-C_pH_{(2p+1-q)}Hal_q$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, N-$(C_1-C_4)$-Alkylsulfamoyl oder N,N-Di-$(C_1-C_4)$-alkylsulfamoyl bedeuten, oder, falls A wie oben unter (a) oder (c) definiert ist, auch gemeinsam $-[CH_2]_n-$ oder -CH=CH-CH=CH- bedeuten können, wobei eine $CH_2$-Gruppe gegebenenfalls durch $NR'$, O, S, SO oder $SO_2$ ersetzt ist, oder einen substituierten $(C_6-C_{12})$-Aryloxy-, $(C_7-C_{11})$-Aralkyloxy, $(C_6-C_{12})$-Aryl- oder $(C_7-C_{11})$-Aralkyl-Rest bedeuten, der im Arylteil 1, 2, 3, 4 oder 5 gleiche oder verschiedene Substituenten aus der Reihe Halogen, Cyano, Nitro, Trifluormethyl, $(C_1-C_6)$-Alkyl, $(C_1-C_6)$-Alkoxy, $-O-[CH_2]_o-C_pH_{(2p+1-q)}Hal_q$, $-OCF_2Cl$, $-O-CF_2-CHFCl$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, $NR'R''$, Sulfamoyl, N-$(C_1-C_4)$-Alkylsulfamoyl oder N,N-Di-$(C_1-C_4)$-alkylsulfamoyl trägt, bedeuten,
$R^3$ Wasserstoff, $(C_1-C_6)$-Alkanoyl, $(C_1-C_6)$-Alkylcarbamoyl, $(C_1-C_6)$-Alkoxycarbonyl, Benzyloxycarbonyl oder eine andere physiologisch verträgliche $N^{im}$-Schutzgruppe bedeutet,
$R^4$ und $R^5$ gleich oder verschieden sind und Wasserstoff oder $(C_1-C_3)$-Alkyl bedeuten,
a) $R^6$ Wasserstoff bedeutet und
$R^8$ Fluor, Chlor oder Brom bedeutet, oder
b) $R^6$ $(C_1-C_3)$-Alkyl bedeutet und
$R^8$ Fluor bedeutet, oder
c) $R^6$ Fluor oder Brom bedeutet und
$R^8$ Wasserstoff bedeutet, oder
d) $R^6$ Fluor bedeutet und
$R^8$ $(C_1-C_3)$-Alkyl bedeutet, oder
e) einer der Substituenten $R^6$ und $R^8$ den Rest $-O-[CH_2]_o-C_pH_{(2p+1-q)}Hal_q$ bedeutet und der andere Wasserstoff, Halogen, $(C_1-C_3)$-Alkyl oder den Rest $-O-[CH_2]_o-C_pH_{(2p+1-q)}Hal_q$ bedeutet, oder
f) einer der Substitutenten $R^6$ und $R^8$ einen substituierten $(C_6-C_{12})$-Aryloxyrest oder $(C_7-C_{11})$-Aralkyloxyrest bedeutet, der im Arylteil 1, 2, 3, 4 oder 5 gleiche oder verschiedene Substituenten aus der Reihe Halogen, Cyano, Nitro, Trifluormethyl, $(C_1-C_6)$-Alkyl, $(C_1-C_6)$-Alkoxy, $-O-[CH_2]_o-C_pH_{(2p+1-q)}Hal_q$, $-OCF_2Cl$, $-O-CF_2-CHFCl$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, Sulfamoyl, N-$(C_1-C_4)$-Alkylsulfamoyl oder N,N-Di-$(C_1-C_4)$-alkylsulfamoyl trägt, bedeutet,
und der andere Wasserstoff, Halogen, $(C_1-C_3)$-Alkyl, den Rest $-O-[CH_2]_o-C_pH_{(2p+1-q)}Hal_q$ oder einen substituierten $(C_6-C_{12})$-Aryloxyrest oder $(C_7-C_{11})$-Aralkyloxyrest bedeutet,
Hal Halogen bedeutet,

$R^7$ Wasserstoff, Halogen, $(C_1-C_{12})$-Alkyl, $(C_1-C_{12})$-Alkoxy, $-O-[CH_2]_o-C_pH_{(2f+1-q)}Hal_q$,$-NR'R''$, $(C_1-C_{12})$-Alkoxy-$(C_1-C_{12})$-alkyl, $(C_1-C_{12})$-Alkoxy-$(C_1-C_{12})$-alkoxy, $(C_7-C_{12})$-Aralkyloxy, $(C_1-C_{12})$-Alkylmercapto, $(C_1-C_{12})$-Alkylsulfinyl oder $(C_1-C_{12})$-Alkylsulfonyl bedeutet, oder
falls mindestens einer der Substituenten $R^6$ und $R^8$ einen substituierten $(C_6-C_{12})$-Aryloxyrest oder $(C_7-C_{11})$-Aralkyloxyrest bedeutet, so kann $R^7$ ebenfalls einen wie vorstehend unter f) definierten substituierten $(C_6-C_{12})$-Aryloxyrest oder $(C_7-C_{11})$-Aralkyloxyrest bedeuten,
$R^5$ und $R^6$ gemeinsam für $-[CH_2]_i-$ stehen,
$R'$ und $R''$ gleich oder verschieden sind und Wasserstoff, $(C_6-C_{12})$-Aryl oder $(C_1-C_{12})$-Alkyl bedeuten oder
$R'$ und $R''$ gemeinsam für $-[CH_2]_h-$ stehen, worin eine $CH_2$-Gruppe durch O, S, N-$(C_1-C_4)$-Alkanoylimino oder N-$(C_1-C_4)$-Alkoxylcarbonylimino ersetzt sein kann,
h = 3, 4, 5 oder 6,
i = 1, 2, 3 oder 4,
n = 3 oder 4 ist,
o = 0, 1, 2 oder 3,
p = 1, 2, 3, 4, 5, 6, 7 oder 8,
q = 0 oder 1 bis (2p + 1) ist,
sowie deren physiologisch verträgliche Salze, mit der Maßgabe, daß die Verbindungen
5-Methyl-2-(2-pyridylmethylthio)-3H-thieno[2,3-d]imidazol,
5-Methyl-2-((4-methoxy-2-pyridyl)methylthio)-3H-thieno[2,3-d]imidazol,
2-(Pyridyl)methylthio-3H-thieno[2,3-d]imidazol,
2-((4-methoxy-2-pyridyl)methylthio)-3H-thieno[2,3-d]imidazol,
5-Acetyl-2-((4-methoxy-3,5-dimethyl-2-pyridyl)methylthio)-3H-thieno-[2,3-d]imidazol,
2-((4-Methoxy-3,5-dimethyl-2-pyridyl)methylthio)-3H-thieno[2,3-d]imidazol-5-carbonsäuremethylester,
5-Acetyl-2-((4-methoxy-2-pyridyl)methylthio)-3H-thieno[2,3-d]imidazol,
5-Acetyl-2-(2-pyridyl)methylthio-3H-thieno[2,3-d]imidazol,
sowie die entsprechenden Sulfinyl-Verbindungen ausgenommen sind.

2. Verbindung der Formel I gemäß Anspruch 1, in welcher A wie in Anspruch 1 unter (b) definiert ist, oder deren physiologisch verträgliche Salze.

3. Verbindung der Formel I gemäß Anspruch 1 oder 2, in welcher T für -SO- steht, oder deren physiologisch verträgliche Salze.

4. Verbindung der Formel I gemäß einem oder mehreren der Ansprüche 1 bis 3, in welcher
$R^1$ und $R^2$ gleich oder verschieden sind und Wasserstoff, $(C_1-C_3)$-Alkyl, Halogen, $(C_1-C_4)$-Alkoxy oder $(C_1-C_4)$-Alkoxycarbonyl bedeuten,
$R^3$ wie in Anspruch 1 definiert ist,
$R^4$ und $R^5$ jeweils Wasserstoff bedeuten,
$R^6$ und $R^8$ gleich oder verschieden und wie in Anspruch 1 unter a) - e) definiert sind und
$R^7$ Wasserstoff, $(C_1-C_3)$-Alkyl oder einen halogenierten Alkoxy-, Alkenyloxy- oder Alkinyloxy-Rest der Formel $-O-[CH_2]_o-C_pH_{(2p+1-q)}Hal_q$,$(C_1-C_7)$-Alkoxy, Benzyloxy oder $(C_1-C_7)$-Alkoxy-$(C_1-C_3)$-alkyl bedeutet, und wobei Halogen vorzugsweise Fluor, Chlor oder Brom bedeutet, o 0 oder 1, p 1 bis 8 und q 0 oder 1 bis (2p + 1) sind, oder deren physiologisch verträgliche Salze.

5. Verbindung der Formel I gemäß einem oder mehreren der Ansprüche 1 bis 4, in welcher
$R^1$ und $R^2$ gleich oder verschieden sind und Wasserstoff oder $(C_1-C_3)$-Alkyl bedeuten,
$R^3$ wie in Anspruch 1 definiert ist,
$R^4$ und $R^5$ jeweils Wasserstoff bedeuten,
$R^6$ und $R^8$ gleich oder verschieden und wie in Anspruch 1 unter a) - e) definiert sind und
$R^7$ Wasserstoff, $(C_1-C_4)$-Alkoxy, einen fluorierten n-Alkoxyrest der Formel $-O-[CH_2]_oC_pH_{(2p+1-q)}Hal_q$ oder $(C_1-C_3)$-Alkyl bedeutet, wobei Hal Fluor bedeutet, o 1, p 1 bis 5 und q 0 oder 1 bis (2p + 1) sind,
sowie deren physiologisch verträgliche Salze.

6. 2-[3-Methoxy-4-(2,2,2-trifluorethoxy)-2-picolylsulfinyl]-1H-thieno[3,4-d]imidazol,
2-[3-Methoxy-4-(2,2,3,3-tetrafluorpropoxy)-2-picolylsulfinyl]-1H-thieno[3,4-d]imidazol,
2-[3-Methoxy-4-(2,2,3,3,3-pentafluorpropoxy)-2-picolylsulfinyl]-1H-thieno[3,4-d]imidazol,
2-[3-Methoxy-4-(2,2,3,3,4,4,4-heptafluorbutoxy)-2-picolylsulfinyl]-1H-thieno[3,4-d]imidazol,
2-(3-Brom-4-methoxy-2-picolylsulfinyl)-1H-thieno[3,4-d]imidazol,
2-(4-Methoxy-5-brom-2-picolylsulfinyl)-1H-thieno[3,4-d]imidazol,
2-(4-Methoxy-5-chlor-2-picolylsulfinyl)-1H-thieno[3,4-d]imidazol,
sowie deren physiologisch verträgliche Salze.

7. Verfahren zur Herstellung einer Verbindung der Formel I, das dadurch gekennzeichnet ist, daß man
a) Verbindungen der Formel II

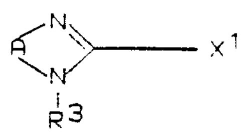

(II)

in welcher A, $R^1$, $R^2$ und $R^3$ wie in Anspruch 1 definiert sind und
$X^1$ i. eine Abgangsgruppe oder
ii. -SH, $-S^-M^+$ oder $-SO_2^-M^+$ bedeutet, wobei $M^+$ für ein Kation steht,
umsetzt mit Verbindungen der Formel III

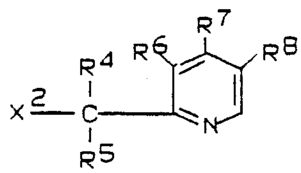

(III)

in welcher $R^4$, $R^5$, $R^6$, $R^7$ und $R^8$ wie im Anspruch 1 definiert sind und
$X^2$ im oben genannten Fall i. -SH, $-S^-M^+$ oder $-SO_2^-M^+$ und
im oben genannten Fall ii. vorzugsweise eine Abgangsgruppe oder OH bedeutet, oder

b) Verbindungen der Formel IV,

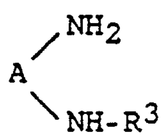

(IV)

in welcher A, $R^1$, $R^2$ und $R^3$ wie im Anspruch 1 definiert sind,
umsetzt mit Verbindungen der Formel V

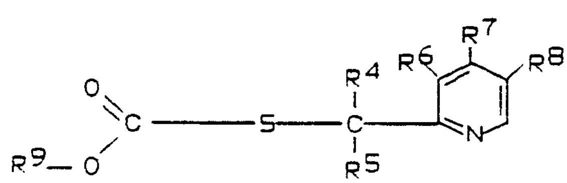

(V)

in welcher $R^4$, $R^5$, $R^6$, $R^7$ und $R^8$ wie im Anspruch 1 definiert sind und $R^9$ für eine veresternde Gruppe steht,
i. in Verbindungen der Formel I (eine) gegebenenfalls vorhandene -S-Gruppe(n) gewünschtenfalls zu(r) -SO- oder - $SO_2$-Gruppe(n) oxidiert,
ii. in Verbindungen der Formel I (eine) gegebenenfalls vorhandene -SO-Gruppe(n) gewünschtenfalls zu(r) -$SO_2$-Gruppe(n) oxidiert,
iii. Verbindungen der Formel I, worin $R^3$ für Wasserstoff steht, gewünschtenfalls acyliert, alkyliert oder aralkyliert,
iv. Verbindungen der Formel I, worin $R^3$ nicht Wasserstoff bedeutet, gewüschtenfalls verseift und
v. Verbindungen der Formel I gewünschtenfalls in ihre physiologisch verträglichen Salze überführt,
wobei zwei oder mehr der Maßnahmen i.-iv. auch in einer anderen als der angegebenen Reihenfolge ausgeführt werden können.

8. Verbindung gemäß einem oder mehreren der Ansprüche 1 bis 6 als Heilmittel.

9. Verbindung gemäß einem oder mehreren der Ansprüche 1 6 als Magensäuresekretionshemmer.

10. Verbindung gemäß einem oder mehreren der Ansprüche 1 bis 6 zur Anwendung als Heilmittel bei der Behandlung entzündlicher Darmerkrankungen.

11. Verbindung gemäß einem oder mehreren der Ansprüche 1 bis 6 zur Anwendung als Heilmittel bei der Behandlung der Diarrhoe.

12. Mittel enthaltend eine Verbindung gemäß einem oder mehreren der Ansprüche 1 bis 6.

13. Verfahren zur Herstellung eines Mittels gemäß Anspruch 12 dadurch gekennzeichnet, daß man eine Verbindung gemäß einem der Ansprüche 1 bis 6, zusammen mit einem physiologisch annehmbaren Träger und gegebenenfalls weiteren Zusatz-, Hilfs- und/oder Konservierungsstoffen in eine geeignete Darreichungsform bringt.

Patentansprüche für die folgenden Vertragsstaaten: ES, GR

1. Verfahren zur Herstellung einer Verbindung der Formel I

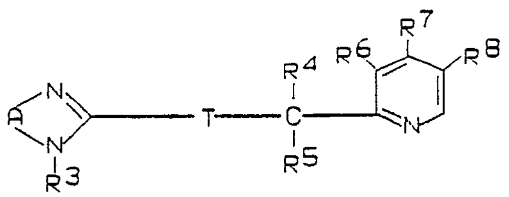

(I)

in welcher

A für a) 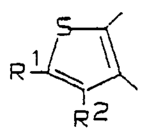 , b) 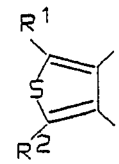 oder c) 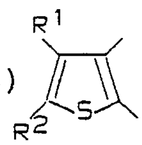 steht,

T -S-, -SO- oder $-SO_2-$ bedeutet,

$R^1$ und $R^2$ gleich oder verschieden sind und Wasserstoff, Halogen, Cyano, Nitro, Trifluormethyl, $(C_1-C_6)$-Alkyl, $(C_1-C_6)$-Hydroxyalkyl, $(C_1-C_6)$-Alkoxy, $-O-[CH_2]_o-C_pH_{(2p+1-q)}Hal_q$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, N-$(C_1-C_4)$-Alkylsulfamoyl oder N,N-Di-$(C_1-C_4)$-alkylsulfamoyl bedeuten, oder, falls A wie oben unter (a) oder (c) definiert ist, auch gemeinsam $-[CH_2]_n-$ oder -CH=CH-CH=CH- bedeuten können, wobei eine $CH_2$-Gruppe gegebenenfalls durch $NR'$, O, S, SO oder $SO_2$ ersetzt ist, oder einen substituierten $(C_6-C_{12})$-Aryloxy-, $(C_7-C_{11})$-Aralkyloxy, $(C_6-C_{12})$-Aryl- oder $(C_7-C_{11})$-Aralkyl-Rest bedeuten, der im Arylteil 1, 2, 3, 4 oder 5 gleiche oder verschiedene Substituenten aus der Reihe Halogen, Cyano, Nitro, Trifluormethyl, $(C_1-C_6)$-Alkyl, $(C_1-C_6)$-Alkoxy, $-O-[CH_2]_o-C_pH_{(2p+1-q)}Hal_q$, $-OCF_2Cl$, $-O-CF_2-CHFCl$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, $NR'R''$, Sulfamoyl, N-$(C_1-C_4)$-Alkylsulfamoyl oder N,N-Di-$(C_1-C_4)$-alkylsulfamoyl trägt, bedeuten,

$R^3$ Wasserstoff, $(C_1-C_6)$-Alkanoyl, $(C_1-C_6)$-Alkylcarbamoyl, $(C_1-C_6)$-Alkoxycarbonyl, Benzyloxycarbonyl oder eine andere physiologisch verträgliche $N^{im}$-Schutzgruppe bedeutet,

$R^4$ und $R^5$ gleich oder verschieden sind und Wasserstoff oder $(C_1-C_3)$-Alkyl bedeuten,

a) $R^6$ Wasserstoff bedeutet und
$R^8$ Fluor, Chlor oder Brom bedeutet, oder
b) $R^6$ $(C_1-C_3)$-Alkyl bedeutet und
$R^8$ Fluor bedeutet, oder
c) $R^6$ Fluor oder Brom bedeutet und
$R^8$ Wasserstoff bedeutet, oder
d) $R^6$ Fluor bedeutet und
$R^8$ $(C_1-C_3)$-Alkyl bedeutet, oder
e) einer der Substituenten $R^6$ und $R^8$ den Rest $-O-[CH_2]_o-C_pH_{(2p+1-q)}Hal_q$ bedeutet und der andere Wasserstoff, Halogen, $(C_1-C_3)$-Alkyl oder den Rest $-O-[CH_2]_o-C_pH_{(2p+1-q)}Hal_q$ bedeutet, oder
f) einer der Substitutenten $R^6$ und $R^8$ einen substituierten $(C_6-C_{12})$-Aryloxyrest oder $(C_7-C_{11})$-Aralkyloxyrest bedeutet, der im Arylteil 1, 2, 3, 4 oder 5 gleiche oder verschiedene Substituenten aus der Reihe Halogen, Cyano, Nitro, Trifluormethyl, $(C_1-C_6)$-Alkyl, $(C_1-C_6)$-Alkoxy, $-O-[CH_2]_o-C_pH_{(2p+1-q)}Hal_q$, $-OCF_2Cl$, $-O-CF_2-CHFCl$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-

Alkoxycarbonyl, Carbamoyl, N-($C_1$-$C_4$)-Alkylcarbamoyl, N,N-Di-($C_1$-$C_4$)-alkylcarbamoyl, ($C_1$-$C_6$)-Alkylcarbonyloxy, ($C_3$-$C_8$)-Cycloalkyl, Sulfamoyl, N-($C_1$-$C_4$)-Alkylsulfamoyl oder N,N-Di-($C_1$-$C_4$)-alkylsulfamoyl trägt, bedeutet,

und der andere Wasserstoff, Halogen, ($C_1$-$C_3$)-Alkyl, den Rest $-O-[CH_2]_o-C_pH_{(2p+1-q)}Hal_q$ oder einen substituierten ($C_6$-$C_{12}$)-Aryloxyrest oder ($C_7$-$C_{11}$)-Aralkyloxyrest bedeutet,

Hal Halogen bedeutet,

$R^7$ Wasserstoff, Halogen, ($C_1$-$C_{12}$)-Alkyl, ($C_1$-$C_{12}$)-Alkoxy, $-O-[CH_2]_o-C_pH_{(2f+1-q)}Hal_q$, $-NR'R''$, ($C_1$-$C_{12}$)-Alkoxy-($C_1$-$C_{12}$)-alkyl, ($C_1$-$C_{12}$)-Alkoxy-($C_1$-$C_{12}$)-alkoxy, ($C_7$-$C_{12}$)-Aralkyloxy, ($C_1$-$C_{12}$)-Alkylmercapto, ($C_1$-$C_{12}$)-Alkylsulfinyl oder ($C_1$-$C_{12}$)-Alkylsulfonyl bedeutet, oder

falls mindestens einer der Substituenten $R^6$ und $R^8$ einen substituierten ($C_6$-$C_{12}$)-Aryloxyrest oder ($C_7$-$C_{11}$)-Aralkyloxyrest bedeutet, so kann $R^7$ ebenfalls einen wie vorstehend unter f) definierten substituierten ($C_6$-$C_{12}$)-Aryloxyrest oder ($C_7$-$C_{11}$)-Aralkyloxyrest bedeuten,

$R^5$ und $R^6$ gemeinsam für $-[CH_2]_i-$ stehen,

$R'$ und $R''$ gleich oder verschieden sind und Wasserstoff, ($C_6$-$C_{12}$)-Aryl oder ($C_1$-$C_{12}$)-Alkyl bedeuten oder

$R'$ und $R''$ gemeinsam für $-[CH_2]_h-$ stehen, worin eine $CH_2$-Gruppe durch O, S, N-($C_1$-$C_4$)-Alkanoylimino oder N-($C_1$-$C_4$)-Alkoxylcarbonylimino ersetzt sein kann,

h = 3, 4, 5 oder 6,

i = 1, 2, 3 oder 4,

n = 3 oder 4 ist,

o = 0, 1, 2 oder 3,

p = 1, 2, 3, 4, 5, 6, 7 oder 8,

q = 0 oder 1 bis (2p + 1) ist,

sowie deren physiologisch verträgliche Salze, mit der Maßgabe, daß die Verbindungen

5-Methyl-2-(2-pyridylmethylthio)-3H-thieno[2,3-d]imidazol,

5-Methyl-2-((4-methoxy-2-pyridyl)methylthio)-3H-thieno[2,3-d]imidazol,

2-(Pyridyl)methylthio-3H-thieno[2,3-d]imidazol,

2-((4-methoxy-2-pyridyl)methylthio)-3H-thieno[2,3-d]imidazol,

5-Acetyl-2-((4-methoxy-3,5-dimethyl-2-pyridyl)methylthio)-3H-thieno-[2,3-d]imidazol,

2-((4-Methoxy-3,5-dimethyl-2-pyridyl)methylthio)-3H-thieno[2,3-d]imidazol-5-carbonsäuremethylester,

5-Acetyl-2-((4-methoxy-2-pyridyl)methylthio)-3H-thieno[2,3-d]imidazol,

5-Acetyl-2-(2-pyridyl)methylthio-3H-thieno[2,3-d]imidazol,

sowie die entsprechenden Sulfinyl-Verbindungen ausgenommen sind, das dadurch gekennzeichnet ist, daß man

a) Verbindungen der Formel II

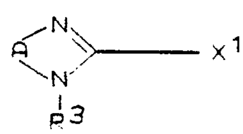

(II)

in welcher A, $R^1$, $R^2$ und $R^3$ wie in Anspruch 1 definiert sind und

$X^1$ i. eine Abgangsgruppe oder

ii. -SH, $-S^-M^+$ oder $-SO_2^-M^+$ bedeutet, wobei $M^+$ für ein Kation steht,

umsetzt mit Verbindungen der Formel III

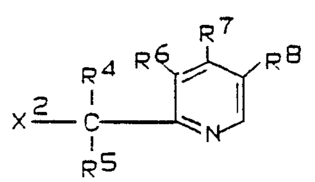

(III)

in welcher $R^4$, $R^5$, $R^6$, $R^7$ und $R^8$ wie im Anspruch 1 definiert sind und

$X^2$ im oben genannten Fall i. -SH, $-S^-M^+$ oder $-SO_2^-M^+$ und

im oben genannten Fall ii. vorzugsweise eine Abgangsgruppe oder OH bedeutet, oder

b) Verbindungen der Formel IV,

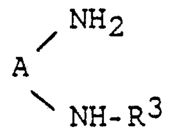

(IV)

in welcher A, $R^1$, $R^2$ und $R^3$ wie im Anspruch 1 definiert sind,
umsetzt mit Verbindungen der Formel V

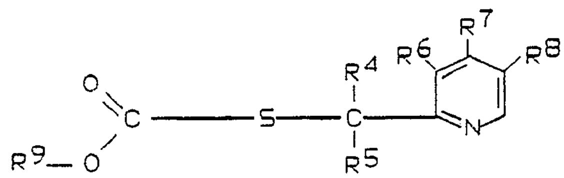

(V)

in welcher $R^4$, $R^5$, $R^6$, $R^7$ und $R^8$ wie im Anspruch 1 definiert sind und $R^9$ für eine veresternde Gruppe steht,

i. in Verbindungen der Formel I (eine) gegebenenfalls vorhandene -S-Gruppe(n) gewünschtenfalls zu(r) -SO- oder - $SO_2$-Gruppe(n) oxidiert,

ii. in Verbindungen der Formel I (eine) gegebenenfalls vorhandene -SO-Gruppe(n) gewünschtenfalls zu(r) -$SO_2$-Gruppe(n) oxidiert,

iii. Verbindungen der Formel I, worin $R^3$ für Wasserstoff steht, gewünschtenfalls acyliert, alkyliert oder aralkyliert,

iv. Verbindungen der Formel I, worin $R^3$ nicht Wasserstoff bedeutet, gewüschtenfalls verseift und

v. Verbindungen der Formel I gewünschtenfalls in ihre physiologisch verträglichen Salze überführt,

wobei zwei oder mehr der Maßnahmen i.-iv. auch in einer anderen als der angegebenen Reihenfolge ausgeführt werden können.

2. Verfahren zur Herstellung einer Verbindung der Formel I gemäß Anspruch 1 oder 2, in welcher A wie in Anspruch 1 unter (b) definiert ist, oder deren physiologisch verträgliche Salze.

3. Verfahren zur Herstellung einer Verbindung der Formel I gemäß Anspruch 1 oder 2, in welcher T für -SO- steht, oder deren physiologisch verträgliche Salze.

4. Verfahren zur Herstellung einer Verbindung der Formel I gemäß einem oder mehreren der Ansprüche 1 bis 3, in welcher

$R^1$ und $R^2$ gleich oder verschieden sind und Wasserstoff, ($C_1$-$C_3$)-Alkyl, Halogen, ($C_1$-$C_4$)-Alkoxy oder ($C_1$-$C_4$)-Alkoxycarbonyl bedeuten,

$R^3$ wie in Anspruch 1 definiert ist,

$R^4$ und $R^5$ jeweils Wasserstoff bedeuten,

$R^6$ und $R^8$ gleich oder verschieden und wie in Anspruch 1 unter a) - e) definiert sind und

$R^7$ Wasserstoff, ($C_1$-$C_3$)-Alkyl oder einen halogenierten Alkoxy-, Alkenyloxy- oder Alkinyloxy-Rest der Formel $-O-[CH_2]_o-C_pH_{(2p+1-q)}Hal_q$, ($C_1$-$C_7$)-Alkoxy, Benzyloxy oder ($C_1$-$C_7$)-Alkoxy-($C_1$-$C_3$)-alkyl bedeutet, und wobei Halogen vorzugsweise Fluor, Chlor oder Brom bedeutet, o 0 oder 1, p 1 bis 8 und q 0 oder 1 bis (2p + 1) sind, oder deren physiologisch verträgliche Salze.

5. Verfahren zur Herstellung einer Verbindung der Formel I gemäß einem oder mehreren der Ansprüche 1 bis 4, in welcher

$R^1$ und $R^2$ gleich oder verschieden sind und Wasserstoff oder ($C_1$-$C_3$)-Alkyl bedeuten,

$R^3$ wie in Anspruch 1 definiert ist,

$R^4$ und $R^5$ jeweils Wasserstoff bedeuten,

$R^6$ und $R^8$ gleich oder verschieden und wie in Anspruch 1 unter a) - e) definiert sind und

$R^7$ Wasserstoff, ($C_1$-$C_4$)-Alkoxy, einen fluorierten n-Alkoxyrest der Formel $-O-[CH_2]_o-C_pH_{(2p+1-q)}Hal_q$ oder ($C_1$-$C_3$)-Alkyl bedeutet, wobei Hal Fluor bedeutet, o 1, p 1 bis 5 und q 0 oder 1 bis (2p + 1) sind,

sowie deren physiologisch verträgliche Salze.

6. Verfahren zur Herstellung von 2-[3-Methoxy-4-(2,2,2-trifluorethoxy)-2-picolylsulfinyl]- 1H-thieno[3,4-d]-imidazol,

2-[3-Methoxy-4-(2,2,3,3-tetrafluorpropoxy)-2-picolylsulfinyl]-1H-thieno[3,4-d]imidazol,

2-[3-Methoxy-4-(2,2,3,3,3-pentafluorpropoxy)-2-picolylsulfinyl]-1H-thieno[3,4-d]imidazol,

2-[3-Methoxy-4-(2,2,3,3,4,4,4-heptafluorbutoxy)-2-picolylsulfinyl]-1H-thieno[3,4-d]imidazol,

2-(3-Brom-4-methoxy-2-picolylsulfinyl)-1H-thieno[3,4-d]imidazol,

2-(4-Methoxy-5-brom-2-picolylsulfinyl)-1H-thieno[3,4-d]imidazol,
2-(4-Methoxy-5-chlor-2-picolylsulfinyl)-1H-thieno[3,4-d]imidazol,
sowie deren physiologisch verträgliche Salze, gemäß Anspruch 1.

7. Verfahren zur Herstellung eines Mittels enthaltend eine Verbindung gemäß einem oder mehreren der Ansprüche 1 bis 6, dadurch gekennzeichnet, daß man eine Verbindung gemäß einem der Ansprüche 1 bis 6, zusammen mit einem physiologisch annehmbaren Träger und gegebenenfalls weiteren Zusatz-, Hilfs- und/oder Konservierungsstoffen in eine geeignete Darreichungsform bringt.

8. Verwendung einer Verbindung gemäß einem oder mehrerer der Ansprüche 1 bis 6 als Heilmittel.